# EUROPEAN PATENT SPECIFICATION

(11) **EP 0 904 090 B1**
(45) Date of publication and mention of the grant of the patent: **03.01.2007**
(21) Application number: 97916061.1
(22) Date of filing: 19.03.1997
(51) Int. Cl.: A61K 35/54

(54) **EGG ANTI-INFLAMMATORY COMPOSITION, METHOD OF ISOLATION AND USE**
ANTI-ENTZÜNDLICH WIRKENDE ZUSAMMENSETZUNG AUS EI, VERFAHREN ZUR ISOLIERUNG UND VERWENDUNG
COMPOSITION ANTI-INFLAMMATOIRE A BASE D'OEUFS, TECHNIQUE D'ISOLATION ET USAGE

(30) Priority: 26.03.1996 US 14097 P; 10.03.1997 US 814187
(43) Date of publication of application: 31.03.1999
(73) Proprietor: Arkion Life Sciences LLC, Wilmington, DE 19810 (US)
(72) Inventor: FITZPATRICK-McELLIGOTT, Sandra, G., Media, PA 19063 (US); HUNCHAR, Jeffrey, G., West Chester, PA 19380 (US); LEE, Young-Zoon, Cincinnati, OH 45249 (US); BECK, Lee, R., Lebanon, OH 45036 (US)
(74) Representative: Carpmaels & Ransford
(86) International application number: PCT/US1997/004328
(87) International publication number: WO 1997/035595

(56) References cited:
- EP-A- 0 472 003
- WO-A-94/09799
- US-A- 5 215 746
- DATABASE WPI Week 9611 Derwent Publications Ltd., London, GB; AN 96-105634 XP002033806 & WO 96 02255 A (TAIYO KAGAKU KK) , 1 February 1996
- PATENT ABSTRACTS OF JAPAN vol. 96, no. 8, 30 August 1996 & JP 08 099988 A (YAMAMOTO TAKEHIKO), 16 April 1996,
- PATENT ABSTRACTS OF JAPAN vol. 95, no. 8, 29 September 1995 & JP 07 126166 A (SAGAMI CHEM. RES. CENTER), 16 May 1995,

## Description

### BACKGROUND OF THE INVENTION

### Field Of The Invention:

The invention generally relates to an anti-inflammatory composition produced in a natural foodstuff. The invention specifically relates to an egg anti-inflammatory composition, methods for its production in substantially and highly purified forms, and methods for its use in the treatment of inflammation.

Inflammation, as defined in Dorland's Medical Dictionary, is "a localized protective response, elicited by injury or destruction of tissues, which serves to destroy, dilute or wall off both the injurious agent and the injured tissue." It is characterized by fenestration of the microvasculature, leakage of the elements of blood into the interstitial spaces, and migration of leukocytes into the inflamed tissue. On a macroscopic level, this is usually accompanied by the familiar clinical signs of erythema, edema, hyperalgesia (tenderness), and pain.

During this complex response, chemical mediators such as histamine. 5-hydroxytryptamine, various chemotactic compositions, bradykinin, leukotrienes, and prostaglandins are liberated locally. Phagocytic cells migrate into the area, and cellular lysosomal membranes may be ruptured, releasing lytic enzymes. All of these events can contribute to the inflammatory response.

Inflammation in patients with rheumatoid arthritis probably involves the combination of an antigen with an antibody complement causing the local release of chemotactic and chemoactivating compositions that attract leukocytes. The leukocytes phagocytose the complexes of antigen-antibody and complement, and also release the many enzymes contained in their lysosomes. These lysosomal enzymes then cause injury to cartilage and other tissues, and this furthers the degree of inflammation. Cell-mediated immune reactions may also be involved. Prostaglandins, which are key intracellular regulators of cellular function, are also released during this process.

The Arthus reaction is an inflammatory response brought about by the formation of immune complexes at subcutaneous sites where an antigen complexes with antibody to that antigen. Neutrophils characteristically attach to the Fc portion of the immunoglobulin complex that forms at the subcutaneous injection site where they release digestive enzymes, causing visible acute inflammation. Thus the reaction is primarily neutrophil-mediated and agents that effect the development of the reaction do so via an effect on these cells.

There are several pathways whereby an agent might interfere with neutrophil migration from the blood vessels to an inflammatory site. One likely pathway is the inhibition of margination, the reversible adhesion or "sticking" of inflammatory cells to the endothelial cell lining of the blood vessel wall. This adhesion is precisely controlled by the quality and quantity of adhesion and integrin molecules that act as "velcro" for white blood cells and the lining of blood vessels. In the normal state about 50% of neutrophils are reversibly adhered, but during an acute inflammatory response, adhesion becomes much stronger and is a key step in the process of neutrophil migration. While prostaglandins are unlikely to be directly involved in the chemotactic response, another product of the metabolism of arachidonic acid, leukotriene, is a very potent chemotactic substance.

The inflammatory response is any response characterized by inflammation as defined above. It is well known, to those skilled in the medical arts, that the inflammatory response causes much of the physical discomfort (i.e., pain and loss of function) that has come to be associated with different diseases and injuries. Accordingly, it is a common medical practice to administer pharmacological agents that reduce the physical discomfort of the inflammatory response. Agents having these properties are classified as anti-inflammatory. Anti-inflammatory drugs are used for the treatment of a wide spectrum of disorders, and the same drugs are often used to treat different diseases. Treatment with anti-inflammatory drugs is not for the disease, but most often for the symptom (i.e., inflammation).

The anti-inflammatory, analgesic, and anti-pyretic drugs are a heterogeneous group of compounds, often chemically unrelated, which nevertheless share certain therapeutic actions and side-effects. Corticosteroids represent the most widely-used class of compounds for the treatment of inflammation. Proteolytic enzymes represent another class of compounds that are thought to have anti-inflammatory effects. Hormones that directly or indirectly cause the adrenal cortex to produce and secrete steroids represent another class of anti-inflammatory compounds.

Unfortunately, the natural and synthetic conicosteroid preparations cause a number of severe side effects, including elevation of blood pressure, salt and water retention, kidney damage and increased potassium and calcium excretion. Moreover, corticosteroids may mask the signs of infection and enhance dissemination of infectious microorganisms. These hormones are considered unsafe for use in pregnant women, and long-term conicosteroid treatment has been associated with gastric hyperactivity and/or peptic ulcers. Treatment with corticosteroids may also aggravate diabetes mellitus, requiring higher doses of insulin, and may produce psychotic disorders. Hormonal anti-inflammatory agents which indirectly increase the production of endogenous corticosteroids have the same potential for adverse side-effects.

A number of non-steroidal, anti-inflammatory agents (NSAID's), have been described. Among these, the most widely used are the salicylates. Acetylsalicylic acid, or aspirin, is the most widely prescribed analgesic-antipyretic and anti-infiammatory agent. Examples of steroidal and non-steroidal ami-inflammatory agents are listed in the Physician's Desk Reference.

The NSAID's are synthetic biochemical compounds which can be toxic at high doses due to a wide spectrum of undesirable side-effects. For example, salicylates contribute to the serious acid-base balance disturbances that characterize poisoning by this class of compounds. Salicylates stimulate respiration directly and indirectly. Toxic doses of salicylates cause central respiratory paralysis as well as circulatory collapse secondary to vasomotor depression. The ingestion of salicylate may result in epigastric distress, nausea, and vomiting. Salicylate-induced gastric bleeding is well-known. Salicylates can produce hepatic injury and prolong clotting time. Therefore, aspirin should be avoided in patients with severe hepatic damage, hypoprothrombinemia, vitamin K deficiency, or hemophilia, because the inhibition of platelet hemostasis by salicylates can result in hemorrhage. Salicylate intoxication is common, and over 10.000 cases of serious salicylate intoxication are seen in the United States every year, some of them fatal, and many occurring in children. See Goodman and Gilman's The Pharmacological Basis of Therapeutics, 7th Ed., 1985.

Accordingly, in spite of the large number of anti-inflammatory agents that are currently available, there still exists a need for a safe, effective anti-inflammatory product that is free of side-effects and adverse reactions.

If a natural food product having anti-inflammatory effects could be obtained, it would provide an easily administratable, readily available, and safe therapeutic composition.

### Related Art:

Milks having a variety of therapeutic effects have been reported. U.S. Patent No. 4.324.782 discloses a milk containing antibody to Streptococcus mutans that has dental caries-inhibiting effects. The milk is obtained by immunizing a cow with S. mutans antigen in two stages. U.S. Patent No. 4.284.623 discloses milk having anti-inflammatory properties. U.S. Patent No. 4.879.110 discloses milk having anti-hypertensive properties. U.S. Patent No. 3.128.230 discloses milk containing globulins of alpha, beta, and gamma components obtained by inoculating a cow with antigenic mixtures. U.S. Patent No. 3,376,198, Canadian Patent 587.849 and British Patent 1,211,876 have also described antibody-containing milks. U.S. Patent No. 4,897,265 and U.S. Patent No. 4,636,384 (Reissue No. 33,403) disclose a method for lowering blood lipid concentrations by feeding animals antibody-containing milk derived from cows maintained in a hyperimmune state by injections of polyvalent bacterial antigens.

EP-A-0727216 describes the preparation of sialic acid derivatives and the present of sialyloligosaccharides in egg yolk. Patent Abstracts of Japan, vol. 95, no.8, 29th September 1995 describes the separation or synthesis of phospholipids from egg yolk.

Various genera of the class Aves, such as chickens (Gallus domesticus), turkeys, and ducks, produce antibodies in blood and eggs against antigens that cause avian diseases, as well as against other antigens. For example. LeBacq-Verheyden et al. (Immunology 27:683 (1974)) and Leslie. G.A., et al. (J. Med. 130:1337 (1969)), have quantitatively analyzed immunoglobulins of the chicken. Polson. A.. et al. (Immunological Communications 9:495-514 (1980)) immunized hens against several proteins and natural mixtures of proteins, and detected IgY antibodies in the yolks of the eggs. Fertel, R., et al. (Biochemical and Biophysical Research Communications 102:1028-1033 (1981)) immunized hens against prostaglandins and detected antibodies in the egg yolk. Jensenius et al. (Journal of Immunological Methods 46:63-68 (1981)) provide a method of isolating egg yolk IgG for use in immunodiagnostics. Polson et al. (Immunological Communications 9:475-493 (1980)) describe antibodies isolated from the yolk of hens that were immunized with a variety of plant viruses.

U.S. Patent No. 4,357,272 discloses the isolation of antibodies from the yolks of eggs derived from hyperimmunized hens. The hyperimmunization was elicited by repetitive injections of antigens derived from plant viruses, human IgG, tetanus antitoxin, snake antivenins, and Serameba. U.S. Patent No. 4.550.019 discloses the isolation from egg yolks of antibodies raised in the hen by hyperimmunization with immunogens having a molecular or particle weight of at least 30.000. The antigens used to hyperimmunize the chickens were selected from among plant viruses, human immunoglobulins, tetanus toxin, and snake venoms.

Additionally, a field study and a controlled infection trial showed the protective effect of antibody egg yolk lyophilisate and immune whole egg lyophilisate against enterotoxic E. coli (Wiedermann et al., J. Vet. Med. B. 38:283-291 (1991)). The orally administered egg yolk powder, prepared from hens vaccinated with heat-killed antigens from K99-piliated enterotoxigenic E. coli, protected neonatal calves against E. coli-induced diarrhea (Ikemori et al., Am. J. Vet. Res. 53:2005-2008 (1992)).

It has also been shown that anti-rotavirus IgY administered to mice infected with rotavirus provides complete protection (Ebina et al., Microbiol. Immunol. 34:617-629 (1990)). Further, egg yolk containing anti-Edwardsiella tarda antibody was found effective in preventing edwardsiellosis in the Japanese eel (Gutierrez et al., J. Fish Diseases 16:113-122 (1993)).

U.S. Patent No. 4,748.018 discloses a method of passive immunization of a mammal that comprises parenterally administering purified antibody obtained from the eggs of an avian that has been immunized against the corresponding antigen, and wherein the mammal has a history of consumption of the eggs. The invention disclosed in U.S. Patent No. 4,748.018 expands on the concepts disclosed in U.S. Patent No. 4.748.018. in that administration of the egg antibody can be by any appropriate route, not only parenteral.

U.S. Application Serial No. 08/688.576. assigned to DCV-Biologics, discloses a method of preventing, countering or reducing chronic gastrointestinal disorders or Non-Steroidal Anti-Inflammatory Drug-induced (NSAID-induced) gastrointestinal damage in a subject by administering hyperimmunized egg and/or milk or fractions thereof to the subject.

Eggs from chickens immunized against specific bacterial antigens were disclosed as providing anti-atherosclerotic effects in mammals in U.S. Patent No. 5,215,746.

Eggs containing antibody to S. mutans were obtained from chickens immunized with S. mutans (Otake et al., J. Dental Research 70:162-166 (1991)) and inhibited dental caries development.

None of these references, however, disclose or suggest that eggs can produce composition(s) and/or factor(s) that can be administered to animals to prevent or reduce inflammation. None disclose or suggest a method providing a reasonable expectation that any treatment of an avian could produce such a composition and/or factor in eggs. None of the references suggest or disclose the identity of an anti-inflammatory component of eggs which produce the desired therapeutic effects.

### SUMMARY OF THE INVENTION

The invention is based on the inventors' discovery that there is anti-inflammatory activity in egg and egg products, and particularly in egg products obtained from hyperimmunized avians, which when administered to a subject, in particular, mammals, prevents or reduces inflammation in the subject.

The invention provides an anti-inflammatory composition in highly purified form, and a method for its preparation, wherein the composition is obtainable from an egg of an egg producing animal by a process comprising (1) isolating a water soluble fraction from the whole egg, egg yolk or egg white; (2) separating a less than 3,000 dalton permeate from the water soluble fraction; and (3) fractionating said less than 3,000 dalton permeate to recover a biologically active fraction.

In particular, the invention is directed to a highly pure anti-inflammatory composition obtained from the eggs of an avian. The anti-inflammatory activity was found in fractions isolated from both egg yolk and egg white.

The invention provides an anti-inflammatory composition in highly purified form obtainable at supranormal levels from an egg of an egg producing animal maintained in a hyperimmunized state by a process comprising:
(1) isolating a water soluble fraction from the whole egg, egg yolk or egg white;
(2) separating a less than 3.000 dalton permeable from the water soluble fraction;
(3) fractionating said less than 3.000 dalton permeate to recover supranormal levels of said anti-inflammatory composition.

The invention is also directed to a highly pure anti-inflammatory composition that is produced by a process which comprises hyperimmunizing an avian by administering to the avian one or more antigens, and especially bacterial antigens, collecting the eggs from the hyperimmunized avian, and isolating the highly pure anti-infiammatory composition therefrom.

The hyperimmunization process produces supranormal levels of a highly pure anti-inflammatory composition from an avian egg. Surprisingly, the level of the anti-inflammatory composition can be increased in both egg yolk and egg white by the process of hyperimmunization.

The invention also describes a method of treating inflammation in a subject, and especially mammals, which comprises administering to the subject the substantially pure anti-inflammatory composition or a composition comprising the substantially pure anti-inflammatory composition. This aspect encompasses administering whole anti-inflammatory egg and/or fractions thereof.

The invention further describes methods for using eggs containing the anti-inflammatory composition and/or the highly purified anti-inflammatory factor itself to prevent lymphocytes and neutrophils from adhering to the endothelium of venules or (to detach lymphocytes and neutrophils which have already adhered to the endothelial cells lining walls of venules) and to reduce the migration of lymphocytes and neutrophils and thus reduce excessive edema and fluid build-up. In this way, the composition is used to reduce the tissue damage associated with the inflammatory response.

The invention finally encompasses a substantially pure anti-inflammatory composition that exhibits anti-inflammatory activity in mammals.

### BRIEF DESCRIPTION OF THE DRAWING

Figure. 1. is a chromatogram showing the separation of the less than 30.000 molecular weight egg anti-inflammatory composition by DEAE Sepharose ion exchange chromatogram.
Figure. 2. is a graph which shows the inhibition of pleural leukocyte migration by the less than 30.000 molecular weight egg anti-inflammatory composition.
Figure 3. is a graph which shows the inhibition of the rat pleural leukocyte migration by endotoxin-free anti-inflammatory composition from egg yolk defatted with hexane-ethanol.
Figure. 4. is a flow chart of the preparation of the less than 30,000 molecular weight anti-inflammatory composition from egg yolk.
Figure 5. is a flow chart of the preparation of the less than 30,000 molecular weight anti-inflammatory composition from liquid egg white.
Figure. 6. is a graph which shows the inhibition of rat pleural leukocytes by endotoxin-free less than 30,000 molecular weight anti-inflammatory composition from S-100 egg yolk defatted by supercritical CO₂ extraction.
Figure. 7. is a flow chart of the preparation of the less than 30,000 molecular weight egg anti-inflammatory composition from dried egg yolk powder.
Figure 8. is a flow chart of the purification of the less than 3,000 molecular weight anti-inflammatory composition from powdered egg yolk.
Figure 9. is a flow chart of the purification of the less than 3,000 molecular weight anti-inflammatory composition from powdered egg white.
Figure 10. is a chromatogram of the separation of the less than 3,000 molecular weight egg anti-inflammatory composition by DEAE Poros ion-exchange chromatography.
Figure 11. is a graph which shows the inhibition of leukocyte migration by less than 3.000 molecular weight highly purified fractions from egg yolk and egg white.
Figure 12. is a graph which shows the inhibition of leukocyte migration by DEAE peak 2, less than 3.000 MW and less than 10.000 MW ultra-filtrates from hyperimmune egg yolk.
Figure 13. is a graph which compares the highly purified less than 3,000 molecular weight anti-inflammatory composition from eggs with the anti-inflammatory drugs (NSAIDS) (Indomethacin and aspirin).
Figure 14. is a chromatogram of the HPLC separation of the less than 3,000 molecular weight anti-inflammatory composition from hyperimmune egg yolk.
Figure 15. is a chromatogram of the HPLC separation of the DEAE fraction from hyperimmune egg yolk.

### DETAILED DESCRIPTION OF THE INVENTION

### Definitions:

The term "egg product" means any product as described herein which contains an anti-inflammatory egg or egg fraction.

The term "anti-inflammatory egg or egg fraction" means egg or egg fractions containing the anti-inflammatory composition disclosed herein, and especially, produced by the processes disclosed herein.

The term "anti-inflammatory composition" means a composition that counteracts or suppresses the inflammatory process.

The term "substantially pure egg anti-inflammatory composition" means an anti-inflammatory composition at least of the purity described in Example 3 and the exemplary materials and figures.

The term "highly pure egg anti-inflammatory composition" means an anti-inflammatory composition at least of the purity described in Example 4 and the exemplary materials and figures.

The term "anti-inflammatory factor" means a substantially pure agent that counteracts or suppresses the inflammatory process which is present in the anti-inflammatory composition at least of the purity described in Example 7.

The term "combinatorial derived antigens" refers to a novel process of generating molecular diversity among antigens by way of combinatorial synthesis.

The term "bioengineered antigens" refers to antigens which are obtained through the process of gene cloning technologies and genetic rearrangements which allow the insertion and translation of proteins which have antigenic properties.

The term "genetic vaccine" refers to a nucleic acid vaccine which is generally produced by recombinant technologies and which may elicit an immune response.

The term "hyperimmunized egg" means whole egg or products derived therefrom, obtained from egg-producing animals maintained in a hyperimmune state.

The term "supranormal levels" means levels in excess of those found in eggs of egg-producing animals not maintained in a hyperimmune state.

The term "table egg" means a non-hyperimmunized egg.

The term "inflammation" is used in its art-recognized sense as a localized protective response elicited by injury or destruction of tissues which serves to destroy, dilute or wall off both the injurious agent and the injured tissue, characterized in the inappropriate, uncontrolled form by the classical sequence of pain, heat, redness, swelling, and loss of function, and histologically involving a complex series of events, including dilation of the arterioles, capillaries, and venules with increased permeability and blood flow, exudation of fluids including plasma proteins, and leukocyte migration into the inflammatory focus.

The term "treating" means that the symptoms of the disorder and/or pathogenic origin of the disorder be prevented, ameliorated or completely eliminated. For example, the anti-inflammatory composition treats inflammation not only by suppressing the symptoms of inflammation in humans and other mammals, but also by acting as a prophylactic agent to counteract the anticipated presence of inflammatory agents in the recipient.

The term "administer" means any method of providing a subject with a substance, including orally, intranasally, parenterally (intravenously, intramuscularly, or subcutaneously) or rectally.

The term "subject" means any living animal that can mount an inflammatory response and is subject to inflammatory disorders and damage, including humans and other animals.

### The Invention

In one embodiment, the invention comprises a highly pure anti-inflammatory composition obtained from avian eggs, its isolation and purification, and the administration of the composition to a subject for the treatment of inflammation. The invention further comprises a highly purified anti-inflammatory composition obtained from the egg of an avian that has been hyperimmunized with one or more antigens, and, in particular, bacterial antigens, or its synthetic equivalent. The highly pure anti-inflammatory composition is present in hyperimmunized eggs at supranormal levels which provide anti-inflammatory activity in subjects.

Although the highly pure anti-inflammatory composition is found in the water-soluble fraction of egg yolk and egg white of any table egg, when isolated from hyperimmunized eggs, the anti-inflammatory composition is found at supranormal levels which are effective in treating inflammation in a subject.

The invention also comprises a substantially pure anti-inflammatory composition obtained from avian eggs, its isolation and purification, and the administration of the composition to a subject for the treatment of inflammation. The invention further comprises a subsantially purified anti-inflammatory composition obtained from the egg of an avian that has been hyperimmunized with one or more antigens, and, in particular, bacterial antigens, or their synthetic equivalent. The substantially pure anti-inflammatory composition is present in hyperimmunized eggs at supranormal levels which provide anti-inflammatory activity in subjects.

The anti-inflammatory composition(s) of the present invention may be administered by any means that provide anti-inflammatory activity. For example, administration may be parenteral, subcutaneous, intravenous, intramuscular, intraperitoneal, intranasal or oral. Solid dosage forms for oral administration include capsules, tablets, pills, powders and granules.

The invention further comprises a substantially pure anti-inflammatory factor which is found within the anti-inflammatory composition. The factor may be administered to a subject by the same means as described above for the anti-inflammatory composition.

The details of the invention are given below.

### Characteristics of the highly purified anti-inflammatory composition:

The highly pure anti-inflammatory composition has the following characteristics:
1) has anti-inflammatory activity in a subject, and in particular, mammals;
2) is present in both the egg white and egg yolk of avian eggs;
3) when isolated from egg yolk, has greater anti-inflammatory activity than when isolated from egg white;
4) has a molecular weight of less than approximately 3000 daltons;
5) is not degraded by enzymes that degrade proteins;
6) is non-proteinaceous and non-steroidal;
7) has a negative charge under prevailing conditions;
8) is heat stable at 100C for at least 30 minutes;
9) is orally active and is not degraded by digestive enzymes:
10) and that is acid and base resistant reverting to an active form even after treatment with conditions of pH 4 to pH 9.0.

The 3.000 dalton molecular weight is deduced from the isolation and purification of the composition wherein the isolation and purification process uses an ultra-filtration membrane that does not allow the passage of molecular species greater than 3.000 Dalton there through. The composition is determined to be non-proteinaceous and non-steroidal because it is small in size and is not degraded by enzymes which degrade proteins. Moreover, the composition is orally active and is not degraded by digestive enzymes. The small stable form of the composition (as differentiated from proteins which are much larger) facilitates its absorption from the digestive tract.

The composition is acid and base resistant, reverting to an active form even after treatment with conditions of pH 9.0. In addition, the composition is heat-stable at 100C for at least 30 minutes and has a negative charge at neutral pH.

The highly purified egg anti-inflammatory composition can be isolated from whole egg, egg yolk and egg white. Anti-inflammatory composition isolated from egg yolk shows higher anti-inflammatory activity than anti-inflammatory composition purified from egg white.

Supranormal levels of the highly purified anti-inflammatory composition can be isolated from hyperimmunized whole egg, egg yolk and egg-white of an avian.

### Characteristics of the substantially purified anti-inflammatory composition:

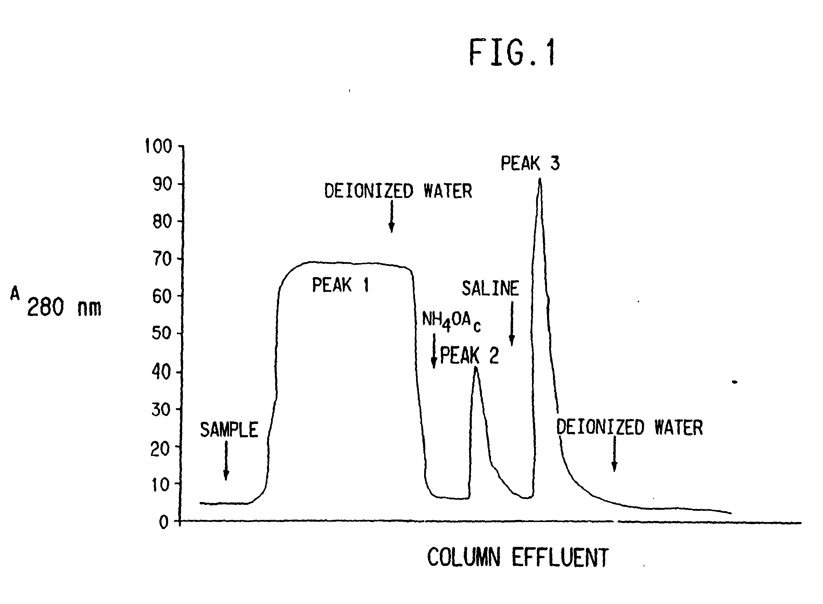

The substantially pure anti-inflammatory composition exhibits anti-inflammatory activity in mammals. The substantially pure anti-inflammatory composition is found in the water-soluble fraction of egg yolk and egg white in the fraction containing molecules of molecular weight less than about 30,000 daltons. The composition has a negative charge, is heat stable at 90C for at least 1 hour, and has the elution profile from egg shown in Figure 1. Furthermore, the composition isolated from egg yolk has a greater anti-inflammatory activity in mammals than factor isolated from egg white.

The molecular weight of the egg anti-inflammatory composition prepared from chicken eggs by the method described above is less than 30,000 Daltons. This was deduced from the fact that the first step in the isolation of the factor from egg yolk and egg white was by ultrafiltration using a membrane that does not allow the passage of molecular weight species greater than 30,000 Daltons. The composition has a negative charge under prevailing conditions, as determined by applying egg 30K ultrafiltrate to a DEAE Sepharose ion exchange column. The anti-inflammatory composition does not elute with water from the column. Changing the elution media to NH₄OAc solution causes the egg anti-inflammatory composition to elute. When the ultrafiltrate is applied to a DEAE Sepharose column, various proteins and salts make up the unbound fraction.

### Hyperimmunization of the Egg-producing Animal:

The invention is based in part on the discovery that when an egg-producing animal such as an avian is brought to a specific state of hyperimmunization, the animal will produce eggs having supranormal levels of the highly beneficial anti-inflammatory composition and thus providing a higher anti-inflammatory effect.

The hyperimmunized egg product can be produced by any egg-producing animal. It is preferred that the animal be a member of the class Aves. Within the class Aves, domesticated fowl are preferred, but other members of this class, such as turkeys, ducks, and geese, are a suitable source of hyperimmunized egg product.

When such egg-producing animals are brought to the specific state of hyperimmunization by means of, for example, periodic booster administration of antigens, the animals will produce eggs that contain supranormal levels of the anti-inflammatory composition which, when administered to a subject, will have beneficial properties in the treatment of inflammation.

The induction of immune sensitivity alone is insufficient to cause the appearance of supranormal levels the egg anti-inflammatory composition in eggs, as is shown by the fact that normal avian's eggs, otherwise referred to as "table eggs." do not contain these supranormal levels, even though the avians have been sensitized against various antigens during normal immunization against avian diseases and during normal exposure to environmental factors. It is only in the specific hyperimmune states that the eggs have the desired supranormal levels.

This special state of hyperimmunization, in which the egg will contain higher levels of the anti-inflammatory composition, is achieved by administering an initial immunization, followed by periodic boosters with sufficiently high doses of specific antigens or mixtures of antigens. The preferred dosage of booster should be equal to or greater than 50% of the dosage necessary to produce primary immunization of the avian. Thus, there is a threshold booster dosage below which the properties are not produced in the avian's egg, even though the avian is in what normally would be called an immune state. Having knowledge of the requirement for developing and maintaining a hyperimmune state, it is within the skill of the art to vary the amount of antigen administered, depending on the egg-producing animal genera and strain employed, in order to maintain the animal in the hyperimmune state.

It should be understood that if levels of the anti-inflammatory composition are increased as described above, then it naturally follows that the levels of the anti-inflammatory factor will correspondingly increase as well.

The hyperimmune composition may be produced by any antigen or combination of antigens. Hyperimmunization may be achieved by multiple exposures to multiple antigens, multiple exposure to single antigens, or single exposures to libraries of immunogens. Nearly any antigen can be used to induce the hyperimmune state, including, but not limited to, bacterial, viral, protozoan, allergan, fungal or cellular substances.

In addition to immunizations with naturally occurring antigens, immunization may also be accomplished using antigens which are synthetically derived by combinatorial chemistries. The basic strategy is to assemble multiple combinations of chemical building blocks for producing a population of molecules with diversity. Several methods have recently been developed for solid and solution phase combinatorial synthesis of libraries of oligomers (Fodor, S. et al., Science 251:767 (1991): Houghton. R. et al., Nature 354:82 (1991)) as well as small organic molecules (Bunin, B. & Ellman, J.. J. Am. Chem. Soc. 114:10997 (1992)). Rapid multiple peptide and oligomer synthesis can serve as a source for combinatorial derived antigens. Furthermore, an alternative strategy would allow the addition of organic building blocks in combinatorial fashion to a backbone molecule for improved antigenicity.

Alternative modes of hyperimmunizing egg producing animals include the use of genetic vaccines or bioengineered antigens. In particular, any DNA construct (generally consisting of a promoter region and an antigen encoding sequence) will trigger antibody release. Genetic vaccines consist of antigen-coding vectors, fragments of naked DNA. plasmid DNA. DNA-RNA antigens. DNA-protein conjugates. DNA-liposome conjugates, DNA expression libraries, and viral and bacterial DNA delivered to produce an immune response.

In particular, evidence now exists that DNA is immunomodulatory as well as immunogenic. Delivery of antigen-encoding vectors, fragments of naked DNA. plasmid DNA. DNA-RNA antigens and DNA expression libraries can in effect mimic live and heat-killed bacterial and viral vaccines. One of the benefits of DNA vaccines is the production and release of antigens from individual cells providing free antigens for the induction of a humoral as well as a cytotoxic immune response. The cytotoxic response is responsible for enhanced effectiveness (Ada. G.L., Lancet 335:523-526 (1990)). An additional advantage is the ability of genetic vaccines to produce foreign proteins with an appropriate three-dimensional conformation.

Genetic immunization is a new approach to vaccine production that has many of the advantages of live/attenuated pathogens but no risk of infection. An expression library comprised of DNA from several sources may be used as vaccines to produce the hyperimmune state.

Methods of DNA delivery include, but are not limited to, particle bombardment, direct injection, liposomes, jet injection (Fynan. E.F. et al., Proc. Natl. Acad. Sci. USA 90:11478-11482 (1993)). The nucleic acids that code for known or unknown immunogens, promoter regions (notably CMV cauliflower mosaic virus) and SV40 bacterial origin can be replicated in bacteria to produce plasmid DNA for use in DNA injections. Although several routes of parenteral administration of the DNA are effective in chickens, the preferred method is intramuscular injection to the breast muscle. Vaccine trials are carried out in egg laying avians, preferably chickens. Repeated immunizations are given at one to two week intervals for up to six months.

It is preferred that the amounts of DNA used are generally in the order of 50-300 µg of DNA in saline for direct injection. For particle bombardment. 4-100 µg of DNA co-precipitated onto gold beads by the addition of 2.5 M CaCl₂ are preferred. Repeated immunizations can be given intradermally by this method of accelerating DNA coated particles into the live animal.

The following is a detailed description of a preferred procedure used to bring an egg-producing animal to a heightened state of immunity from which the resultant hyperimmunized egg or egg product can be administered to a subject:
1. Selecting one or more antigens.
2. Eliciting an immune response in the egg-producing animal by primary immunization.
3. Administering booster vaccines of antigens of appropriate dosage to induce and maintain the hyperimmune state.
4. Testing the hyperimmunized eggs for anti-inflammatory activity levels.
5. Collecting and processing the eggs.

The following is a more detailed description of this procedure.

Step 1: Any antigens or combination of antigens may be employed. The antigens can be bacterial, viral, protozoan, fungal, cellular, allergan or any other substance to which the immune system of an egg-producing animal will respond. The critical point in this step is that the atitigen(s) must be capable, not only of inducing immune and hyperimmune states in the egg-producing avian, but also of inducing the production of the anti-inflammatory composition in the egg. One preferred vaccine is a mixture of polyvalent bacterial antigens, referred to as Series 100 (S-100) vaccine. The bacteria included in the S-100 vaccine are listed in table 1 of Example 1. This vaccine has been previously described in U.S. Patent Nos. 5.106,618 and 5.215,746, both assigned to Stolle Research and Development Corporation.

Step 2: The vaccine can be administered by any method that elicits an immune response. It is preferred that immunization be accomplished by administering the antigens through intramuscular injection. The preferred muscle for injection in an avian is the breast muscle. Dosage is preferably 0.5 - 5 milligrams of the antigen(s) vaccine. Other methods of administration that can be used include intravenous injection, intraperitoneal injection, rectal suppository, and oral administration, among others. When DNA techniques are used for the hyperimmunization process, much smaller quantities are required, generally 1 300 micrograms.

It can be determined whether the vaccine has elicited an immune response in the egg-producing animal through a number of methods known to those of skill in the art of immunology. Examples of these include enzyme-linked immunosorbent assays (ELISA), tests for the presence of antibodies to the stimulating antigens, and tests designed to evaluate the ability of immune cells from the host to respond to the antigen. In general, the appearance of egg antibodies after immunization with the vaccine is indicative of an immune response. The minimum dosage of antigen necessary to induce an immune response depends on the vaccination procedure used, including the type of antigen(s) used as well as the type of egg-producing animal used as the host.

Step 3. The hyperimmune state is preferably induced and maintained by repeated booster administration of an appropriate dosage at fixed time intervals. The time intervals are preferably two-week intervals over a period of six months. It is essential that the booster administrations do not lead to immune tolerance.

It is also possible to use other hyperimmunization maintenance procedures or combination of procedures, such as, for example, intramuscular injection for primary immunization and intravenous injection for booster injections. Further procedures include simultaneously administering microencapsulated and liquid antigen, or intramuscular injection for primary immunization, and booster dosages by oral administration or parenteral administration by microencapsulation means. Several combinations of primary and hyperimmunization as known to those skilled in the art.

Step 4. It is necessary to test the eggs for anti-inflammatory activity levels. This can be accomplished by any clinical and pre-clinical evaluation that tests the effects of either the hyperimmune egg, or products derived therefrom, on inflammation. Chemical-induced inflammation of the rat is a standard assay for anti-inflammatory drugs. (See Example 6a,b,c).

Step 5. This step involves the collection and processing of the egg(s) containing the anti-inflammatory composition. The egg can be collected by conventional methods. Processing the egg to isolate and purify the anti-inflammatory composition is described below.

### Isolation and Purification:

The isolation and purification of the egg anti-inflammatory composition can be accomplished using whole egg, egg yolk or egg white. An example of a preferred process is as follows:
1. Preparation of a water-soluble fraction from an egg;
2. Ultrafiltration of the water-soluble fractions;
3. Separation of fractions by reverse phase High Pressure Liquid Chromatography; and
4. Bioassay of the separated fractions for anti-inflammatory activity.
The following is a more detailed description of this process:

### Step 1:

The anti-inflammatory composition can be isolated from whole egg, egg yolk or egg white. In a preferred embodiment, the composition is isolated by this method from egg white. In an especially preferred embodiment, the composition is isolated from egg yolk. The lipid portion is removed from the whole egg or egg yolk by methods well-known to those having skill in the art. For example, in the case of spray-dried egg yolk powder, defatting can be accomplished with solvents (propane, butane or hexane or with binary solvents), supercritical CO₂, enzymes and the like, and in the case of liquid egg yolk, defatting can be accomplished by the caprylic acid separation method (CAPS) disclosed by Lee (U.S. Pat. No. 5.367.054). No fat removal is necessary for egg white, and thus the liquid or powdered form of the egg white can be either heated or dissolved directly by conventional methods and as described in the examples listed below. The whole egg, egg yolk or egg white is then preferably processed into either liquid or powder form, and is further processed to obtain water soluble fractions. (See Examples)

### Step 2:

The resulting water soluble fractions, from whole egg, egg yolk or egg white, are subjected to ultrafiltration usine ultrafiltration systems equipped with a 3,000. molecular weight cut-off membrane. The ultrafiltration process separates molecules having a molecular weight of more than 3.000 daltons from those having a molecular weight of less than 3000 daltons. Once filtered, the resulting ultra-filtrates contain molecules of less than 3,000 dalton molecular weight are then lyophilized, weighed, and prepared for bioassay testing. In a specific disclosed embodiment, the preferred ultrafiltration is by Amicon RA1000 (3K MWCO) and DEAE ion-exchange chromatography. It is understood, however, that equivalent techniques and materials could be used to isolate the composition, given the information herein, and the knowledge available to the person of ordinary skill in the art.

### Step 3:

Fractions from the less than 3.000 dalton ultra-filtrate can be separated by, for example. Reverse Phase High Pressure Liquid Chromatography to isolate the highly pure anti-inflammatory composition. As an alternate or additional strategy, the egg anti-inflammatory composition in the ultra-filtrate can be further characterized by anion exchange DEAE-Sepharose chromatography. Such methods of separation are well known by those having ordinary skill in the art.

### Step 4:

The anti-inflammatory activity of the composition can be tested by any bioassay which determines anti-inflammatory activity. Some examples include inhibition of leukocyte migration, rat paw edema test adjuvant-induced arthritis, collagen induced arthritis, and intra-vital microscopy among others. Comparisons of egg anti-inflammatory composition with known anti-inflammatory drugs such as aspirin and indomethacin can also be done. And finally, clinical tests for rheumatoid arthritis, degenerative joint disease and injury induced arthritis can also be used to determine anti-inflammatory activity.

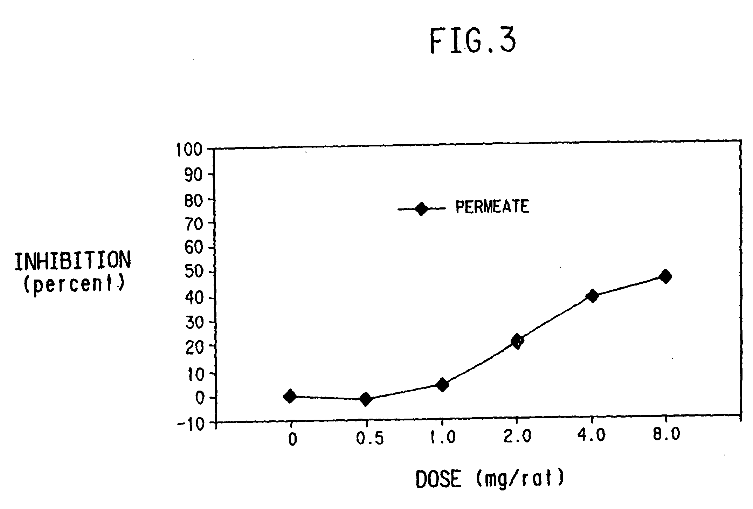

The anti-inflammatory action of the highly pure egg anti-inflammatory composition is measured by bio-assavs. A preferred bio-assay is the pleural leukocyte migration inhibition assay as described in Vinegar et al., "Some Quantitative Characteristics of Carrageenan Induced Pleurisy in the Rat," Proc. Soc. Exp. Bio. Med. 143:711-714 (1973); Ammendola, G. et al., "Leukocyte Migration and Lysozomal Enzymes Release in Rat Carrageenan Pleurisy." Agents and Actions 5:250-255 (1975): Vinegar. R. et al., "Quantitative Studies of the Pathway to Acute Carrageenan Inflammation." Fed. Proc. 35:2447-2456 (1976) (see Examples 7, 7a, 7b, 7c and 9, Figs. 3, 5 and 6).

The leukocyte migration inhibition assay is generally performed as follows: Samples containing the anti-inflammatory composition are administered to the artificially inflamed adult female rats with 1% carrageenan solution and then the anti-inflammatory effect of the samples at each dosage is determined (using Automated Image Analysis Technique) by the reduction in the number of leukocytes in the pleural exudates of the treated rats as compared to those of control rats.

Alternatively, the anti-inflammatory action of the substantially pure anti-inflammatory composition can be tested on edema caused by injecting carrageenan into rat footpads (Winter, C.A.. Risley. G.A.. Nuss. A.W., "Carrageenan-Induced Edema in the Hind Paw of the Rat as an Assay for Anti-Inflammatory Drugs." Proc. Soc. Exper. Biol. Med. 3:544 (1967)).

A variety of other tests may be used. (See Wetnick. A.S., and Sabin. C., "The Effects of Clonixin and Bethaurethasone on Adjuvant-Induced Arthritis and Experimental Allergic Encephalomyelitis in Rats." Jap. J. Pharm. 22:741 (1972)).

### Administration to a Subject for Treatment:

The invention is also directed to a method of treating inflammation in a subject which comprises administering to the subject the egg, egg product and/or the anti-inflammatory composition of this invention.

The anti-inflammatory composition of the present invention may be administered by any means that provide anti-inflammatory activity. For example, administration may be parenteral, subcutaneous, intravenous, intramuscular, intraperitoneal, intranasal, or oral.

Oral administration is preferably accomplished through solid dosage forms which include capsules, tablets, pills, powders and granules, among others. In solid dosage forms, the anti-inflammatory composition is admixed with at least one inert diluent such as sucrose, lactose or starch. Such dosage forms can also comprise, as is normal practice, additional substances other than inert diluent. In the case of capsules, tablets, and pills, the dosage forms may also comprise buffering agents, pH sensitive polymers, or any other slow-releasing encapsulants which are typically used as encapsulating compositions in the food and drug industry. Tablets and pills can additionally be prepared with an enteric coating.

Liquid dosage forms of the anti-inflammatory composition for oral administration include pharmaceutically acceptable emulsions, solutions, suspensions, syrups and elixirs, containing inert diluents commonly used in the pharmaceutical art.
Besides inert diluents, compositions can also include wetting agents, emulsifying, and suspending , and sweetening agents.

Preparations of the anti-inflammatory composition for parenteral administration include sterile aqueous or nonaqueous solutions, suspensions or emulsions. Examples of nonaqueous solvents or vehicles are propylene glycol, polyethylene glycol, vegetable oils such as olive oil and injectable organic esters such as ethyl oleate.

The dosage of active ingredients may be varied: however it is necessary that the amount of the active ingredient shall be such that a suitable dosage form is obtained. It will be recognized that the selected dosage form depends upon the desired therapeutic effect, on the route of the administration and on the duration of the treatment.

It is preferred that the egg itself, which contains the anti-inflammatory composition, be incorporated into a food product to form the egg product. One preferred method for preparing the egg to be incorporated into a food product involves drying the egg into an egg powder. Although various methods are known for drying eggs, spray drying is a preferred method. A temperature of no more than 140 F (60 C) is preferably used. Samples are monitored for moisture content during the drying process to obtain a final product having any consistency desired. The dried egg powder can be used in drinks, protein supplements and any other nutritional, athlete-associated products. In addition, the egg powder can be used in bake mixes, powder bars, candies, cookies, etc. Other examples of egg processing include, making an omelet, soft or hard-boiling the egg, baking the egg, or, if desired, the egg can be eaten raw.

Administration dosage and frequency will depend on the age and general health condition of the patient, taking into consideration the possibility of side effects. Administration will also be dependent on concurrent treatment with other drugs and patients' tolerance of the administered drug.

The preferred mode of storage for preparations after the ion-exchange step is as a lyophilized powder. The filtrate collected in the first purification step may be stored refrigerated until use. The activity of the anti-inflammatory composition resulting from the purification is determined using the rat pleural leukocyte migration test described previously.

Examples of inflammatory conditions that may be treated by administration of the egg, egg product, and/or the anti-inflammatory composition of the present invention include acute and subacute bursitis, acute non-specific tendinitis, systemic lupus erythematosus, systemic dermatomyositis, acute rheumatic carditis, pemphigus, bullous dermatitis, herpeteformis, severe erythema, multiform exfoliative dermatitis, cirrhosis, seasonal perennial rhinitis, bronchial asthma, ectopic dermatitis, serum sickness, keratitis, opthalmicus iritis, diffuse ureitis, chorditis, optic neuritis, sympathetic ophthalmia, symptomatic sarcoidosis, Loeffler's syndrome, berylliosis, hemolytic anemia, mastitis, mastoiditis, contact dermatitis, allergic conjunctivitis, psoriatic arthritis, ankylosing spondylitis, acute gouty arthritis, herpes zoster rheumatoid arthritis, osteoarthritis, any other degenerative joint diseases, and any other related autoimmune diseases. Further, the isolated and purified egg product may be used to treat individuals who are exposed to potentially inflammatory agents such as allergans.

### Effective amounts:

With regard to administration to a subject of the hyperimmunized egg or egg product, it has been determined, and is detailed in the following examples, that the preferred dose range of hyperimmunized egg or egg product to be given to a subject is between 1 gram to 40 grams per kilogram of subject weight.

With regard to the highly purified anti-inflammatory composition itself, it has been determined that the preferred dose range of the highly purified composition, purified and isolated from whole egg, egg yolk and egg white of a hyperimmunized egg, is between 1 microgram and 400 milligrams of the anti-inflammatory composition.

### Anti-inflammatory Factor:

Once the anti-inflammatory composition is isolated and purified, active fractions can be further purified and examined, with regard to both structure and activity to determine characteristics of a more specific, active anti-inflammatory compound In particular, using high pressure liquid chromatography (HPLC), a single, ultra-pure peak has been identified which shows anti-inflammatory activity. It is believed that this single, ultra-pure peak represents a single compound having an elemental chemical structure. This compound has been labeled the egg "anti-inflammatory factor."

Having now generally described this invention, the same will be further described by reference to certain specific examples which are provided herein for purposes of illustration only and are not intended to be limiting unless otherwise specified.

### EXAMPLES

### Example 1

### Preparation of S-100 Vaccine

The multivalent vaccine known as "Series 100" or "S-100." disclosed in U.S. Pat. No. 5,215,746 and containing the bacteria shown in Table 1 (obtained from the American Type Culture Collection), was reconstituted with 15 ml of medium and incubated overnight at 37C. Once good growth was obtained, approximately one-half of the bacterial suspension was used to inoculate one liter of broth which was then incubated at 37C. The remaining suspension was transferred to sterile glycol tubes and stored at -20C for up to six months.

After good growth was visible in the culture, the bacteria were harvested by centrifugation. The bacterial pellet was resuspended in sterile saline solution and the bacterial sample was centrifuged three times to wash the cells. After the third wash, the pellet obtained was resuspended in a small amount of double distilled water.

The medium-free bacterial suspension was heat-killed by placing the suspension in a glass flask in an 80C water bath overnight. The viability of the broth culture was tested with a small amount of heat-killed bacteria. Broth was inoculated with heat-killed bacteria, incubated at 37C for five days and checked daily for growth, as the bacteria have to be killed for use in the vaccine.

The heat-killed bacteria were lyophilized until dry. The dry bacteria were then mixed with sterile saline solution to a concentration of 2.2 x 10⁸ bacterial cells/ml saline (1.0 optical density reading at 660 nm).

**TABLE 1**

| Antigens in the S-100 Vaccine | | | |
|---|---|---|---|
| **Name** | **Media** | **Name** | **Media** |
| Staphylococcus simulans | BHI | Pseudomonas aeruginosa | BHI |
| Staphylococcus epidermidis | BHI | Klebsiella pneumoniae | BHI |
| Streptococcus pyogenes, A Type 1 | APT | Salmonella typhimurium | BHI |
| Streptococcus pyogenes, A Type 3 | APT | Haemophilus influenzae | BHI |
| Streptococcus pyogenes, A Type 5 | APT | Streptococcus mitis | APT |
| Streptococcus pyogenes. A Type 8 | APT | Proteus vulgaris | BHI |
| Streptococcus pyogenes. A Type 12 | APT | Shigella dysenteriae | BHI |
| Streptococcus pyogenes. A Type 14 | APT | Diplococcus pneumoniae | APT |
| Streptococcus pyogenes. A Type 18 | APT | Propionibacter acnes | Broth |
| Streptococcus pyogenes. A Type 22 | APT | Streptococcus sanguis | APT |
| Aerobacter aerogenes | BHI | Streptococcus salivarus | APT |
| Escherichia coli | BHI | Streptococcus mutans | BHI |
| Salmonella enteritidis | BHI | Streptococcus agalactiae | APT |

### Example 2

### Anti-inflammatory effect of Hyperimmunized Egg

This example shows the anti-inflammatory effect of hyperimmunized egg, as described in Example 1. on carrageenan induced skin edema in dogs on a diet including hyperimmunized egg. These results demonstrate that hyperimmune egg reduces inflammation. The effect on inflammation was comparable to that obtained with the non-steroidal anti-inflammatory drug ibuprofen at 10 mg /kg. ED₅₀ dose in this test.

Twenty White Eagle Beagles (four groups of five dogs each) were fed a basal diet (350 grams) of commercially available lamb and rice dog food. As illustrated in table 2, two groups of five dogs (groups 1 & 2) received only the basal diet during the conditioning period of approximately 100 days. In addition, dogs from group 2 were treated with the non-steroidal anti-inflammatory drug, ibuprofen, prior to inflammatory challenge with carrageenan. During this same period, the remaining two groups (3&4) were fed the basal diet along with hyperimmune egg (HIE). Group 3 received 3.5 grams of hyperimmune egg, while group 4 received 35 grams of hyperimmune egg added to the diet. At the end of the conditioning diet treatment, the dogs were challenged with an intra-dermal injection of 2% carrageenan and an inflammatory response was elicited.

The challenge process was as follows. One dog from each group was randomly selected for challenge and testing each day. This procedure was repeated daily for five days until all dogs from all groups were tested. Fifteen minutes prior to the challenge procedure, dogs from group 2 were given 10mg/kg ibuprofen orally. All dogs were anesthetized by intravenous injection and shaved on the left lateral side (approximately 6 X 8 inches). Two lines of 3 marks were made on the shaved area and numbered 1 - 6. An intra-dermal injection of 0.1 ml saline was given at mark 1 as a negative control. From mark 2 - 6 , a 0.1 ml intra-dermal injection of a 2% solution of carrageenan was given. Injections were administered by the same person for the duration of the study. Intra-dermal injections of this concentration of carrageenan was previously determined to cause a measurable swelling in 100% of the animals which could be reduced by 10 mg/kg ibuprofen. At the completion of the last injection of carrageenan, measurements were made of each swelling using a micrometer and the numbers were recorded. The anesthetized animals were allowed to recover and six hours later the measurements were repeated and recorded. The mean size of the inflammatory response was determined for each group.

**TABLE 2**

| Groups | Treatment | Number of Animals Per Group | Mean Inflammatory Response | Mean Difference from Control | Significance |
|---|---|---|---|---|---|
| | | | | | P value |
| 1 Control | Basal Diet | 5 | 4.41 | - | - |
| 2 Drug | Ibuprofen | 5 | 2.25 | 2.2 | P<0.01 |
| | (10mg/kg)⁺ Basal Diet | | | | |
| 3 Test | HIE egg | 5 | 2.8 | 1.6 | P<0.05 |
| | 3.5 grams + Basal Diet | | | | |
| 4 Test | HIE egg | 5 | 2.38 | 2.0 | P<0.05 |
| | 35 grams ⁺ Basal Diet | | | | |

These results showed a significant reduction in swelling in the animals fed with hyperimmune egg (3.5 grams plus basal diet and 35 grams plus a basal diet) indicating a preventative effect of the hyperimmune egg on inflammation when fed orally. Moreover these results show that the anti-inflammatory activity of the hyperimmune egg was comparable to the effect seen with the anti-inflammatory drug ibuprofen. Differences at the p<0.05 level of significance were seen between the treated and control group.

### Example 3

### Isolation of <30.000 Dalton Egg Anti-infiammatory Composition from Hyperimmune Avian Egg

### Step 1: Preparation of Water-Soluble Filtrate from Egg

Liquid egg yolk, liquid egg white, and egg yolk powder was processed to obtain the water-soluble fraction. Liquid egg yolk was defatted by the caprylic acid phase-separation method (CAPS) disclosed by Lee (U.S. Pat. No. 5.367,054) as described in these examples. Liquid egg white was heated for a specified length of time as described in these examples. Egg yolk powder was defatted with binary solvent, supercritical CO₂, or enzyme, as described in these examples.

### Step 2: Ultrafiltration of the Water-Soluble Fractions

The water-soluble fractions were concentrated in an Amicon DC10L Ultrafiltration System with a spiral-wound 30K MWCO ultrafiltration membrane. The ultrafiltration cartridge has a membrane surface area of 10ft², and was run near 40 psi inlet and 33 psi outlet pressure (7 psi transmembrane pressure) with a recirculation flow rate of 10.4 L/min and a flux flow rate of 2.3 L/min.

### Step 3: Ion-Exchange Chromatography

The filtrate from the DC10L ultrafiltration of the water-soluble fractions was further processed by anion exchange chromatography. In this procedure. DEAE-Sepharose Fast Flow gel (Pharmacia) was used to pack a 3 x 15 cm glass column which was equilibrated with sterile double distilled water, pH 7.0.

Five hundred ml of the filtrate (<30.000 daltons), prefiltered through a 0.2 micron filter, was applied to the column which was washed with 500 ml of sterile, double distilled water, pH 7.0. at the flow rate 10 ml per min. The bound fraction, suspected to contain egg anti-inflammatory composition, was eluted with 250 ml of 0.15M NH₄OAc. The fractions were collected and monitored at 280 nm in an LKB Uvicord 2138 monitor with optical density printed out on a connected recorder. The elution profile of endotoxin-free substantially pure egg anti-inflammatory composition is shown in Figure 1. The egg anti-inflammatory composition fraction was lyophilized for further processing.

### Preparation of < 30.000 Dalton Egg Anti-inflammatory Composition From Liquid Egg Yolk and Liquid Egg White

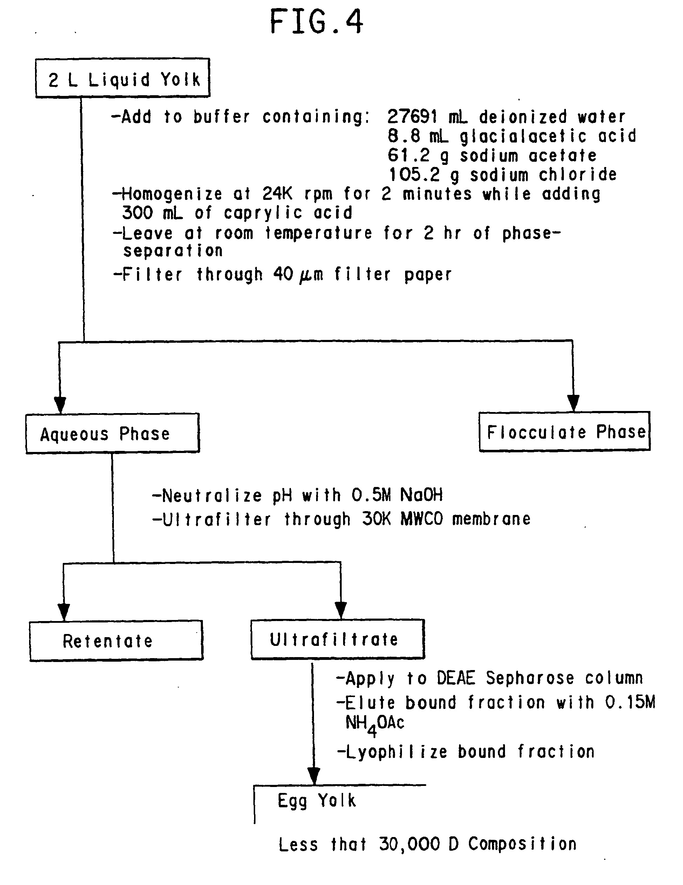

Two liters of hyperimmune egg yolk was defatted by the caprylic acid phase-separation method (CAPS) disclosed by Lee (U.S. Pat. No. 5,367,054). Briefly, S-100 egg yolk was diluted 15-fold in a buffered solution of 1% caprylic acid and 0.06M sodium chloride in 0.02M acetate, at a pH of 5.0. The aqueous phase was separated by filtration from the flocculate phase, and adjusted to pH 7 with 0.5M NaOH. The aqueous phase was ultrafiltered until 2 L of retentate was left. The ultrafiltrate was applied to the DEAE Sepharose column. 46.6 g of egg yolk anti-inflammatory composition was obtained from 2 L of egg yolk after DEAE column chromatography (see Figure 4).

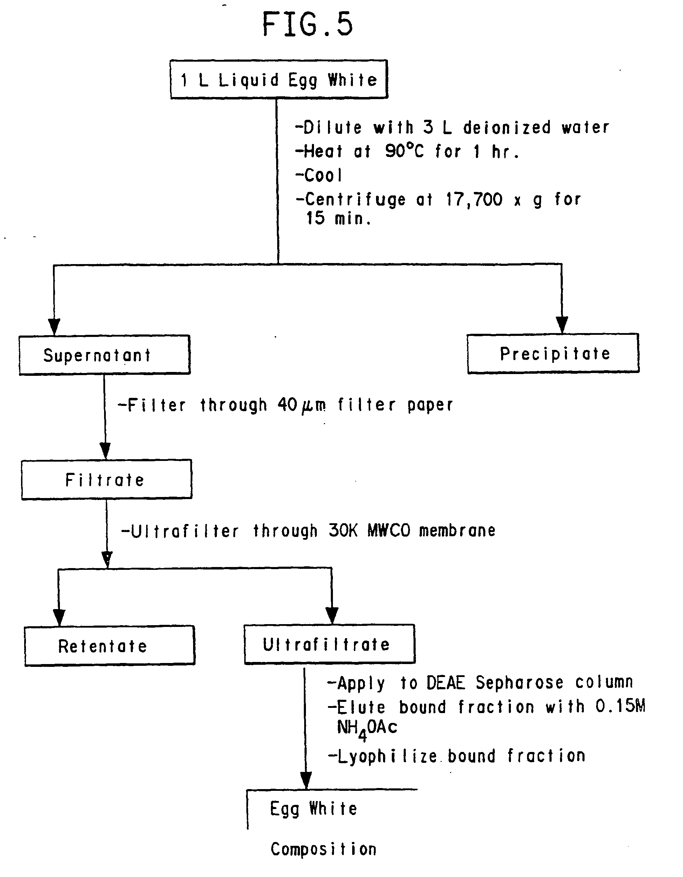

One liter of hyperimmune egg white was diluted with 3 L of deionized water. The mixture was heated to 90C for 1 hour, cooled, and centrifuged. The supernatant was filtered through 40 micron filter paper and ultrafiltered until approximately 64 ml of retentate was obtained. Five hundred ml of ultra filtrate was applied to the DEAE Sepharose column. From 1 L of egg white. 4.4 g of anti-inflammatory composition was obtained after DEAE Sepharose column chromatography. (See Figure 5).

### Preparation of <30.000 Dalton Egg Anti-inflammatory Composition From Egg Yolk Powder

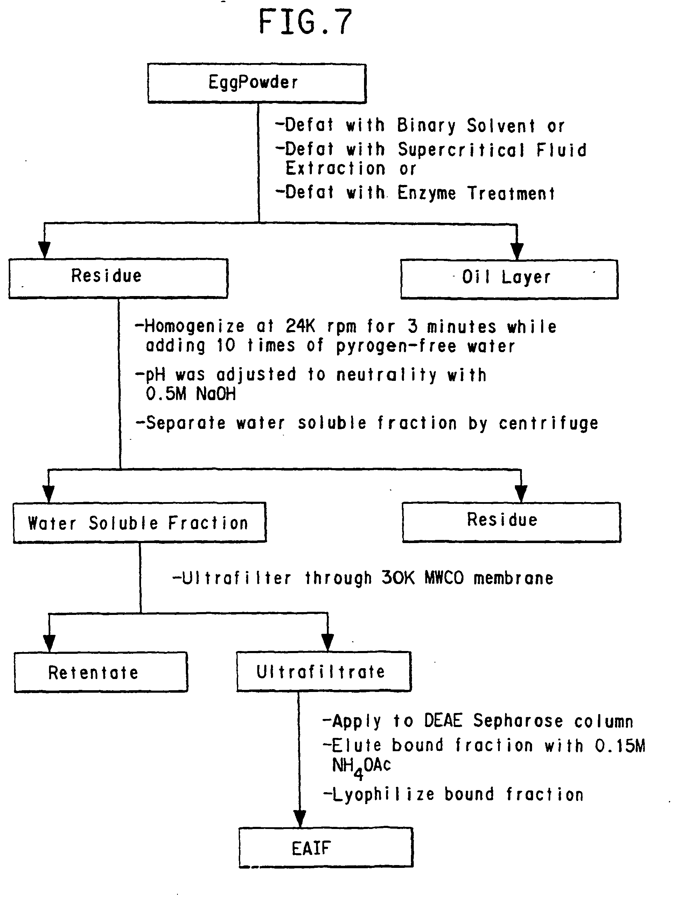

Three procedures were used to defat egg yolk powder and then isolate egg anti-inflammatory composition from the defatted egg yolk powder (Figure 7).

### Procedure 1: Binary Solvent Extraction

Hyperimmune egg yolk powder was combined with a mixture consisting of absolute ethanol and hexane to remove lipids from yolk, followed by aqueous separation of the egg anti-inflammatory composition fraction. Briefly. 400 g of hyperimmune yolk powder was combined with 2 L of binary solvent (25% absolute ethanol-75% hexane or 25% iso-propanol-75% hexane) and mixed. The solvent supernatant was removed, and two additional solvent extractions were performed for a total of three lipid extractions. The final extraction mixture was centrifuged to aid in final separation. The defatted yolk material was dried, and combined with 4.000 ml deionized water, and pH was adjusted to 7 with 0.5M NaOH. It was then concentrated to 500 ml in an Amicon DC10L Ultrafiltration-System.

The egg anti-inflammatory composition in the ultrafiltrate (<30,000 daltons) was further isolated by DEAE-Sepharose chromatography, and the egg anti-inflammatory composition fractions were lyophilized for further processing. After DEAE chromatography. 0.45g anti-inflammatory composition was recovered from the ultrafiltrate and.tested for activity (Figure 3).

### Procedure 2: Supercritical CO₂ Extraction

Dry hyperimmune yolk powder was placed under supercritical conditions to remove a portion of the lipids from yolk, followed by aqueous separation of egg anti-inflammatory composition fraction. In summary, approximately 400 g of dry immune yolk powder was placed in the Autoclave Engineers® SCE unit, and allowed to undergo static CO₂ extraction at -5400 psi at 32,2C for 20 hours. The partially-defatted yolk was homogenized with 4.000 ml deionized water and centrifuged at 10.000 rpm for 30 minutes. The aqueous supernatant was separated and then concentrated to 500 ml in an Amicon DC10L Ultrafiltration System. The egg anti-inflammatory composition in the permeate (<30,000 daltons) was further isolated by DEAE-Sepharose anion exchange chromatography.

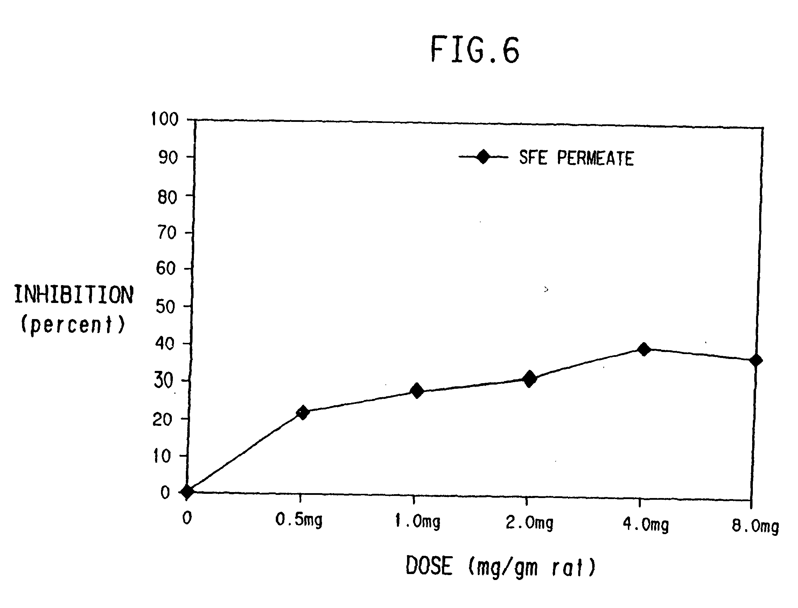

The egg anti-inflammatory composition fractions were lyophilized for further processing, and 1.86 g of egg anti-inflammatory composition was obtained from the ultrafiltrate after DEAE chromatography. Figure 6 shows the bioassay activity of the substantially pure composition after defatting by supercritical CO₂ extraction, ultrafiltration and DEAE chromatography.

### Procedure 3: Enzyme Treatment Extraction

Hyperimmune egg yolk powder was incubated with a mixture consisting of Newlase F in lactate buffer to separate lipids from yolk, followed by aqueous separation of the anti-inflammatory composition fraction. Briefly. 300 grams of dried S-100 egg yolk powder was mixed with 6 L of 0.05M lactate buffer, pH 4.0, and 27 g of Newlase F (Sigma #P-5027) and homogenized. The mixture was incubated at 50°C for one hour and then the bottom aqueous phase was filtered. The resulting aqueous phase was neutralized with 0.5M NaOH and then concentrated on Amicon DC10L (30K MWCO) until 3 L of permeate was collected. 276 ml of the permeate was applied to a DEAE Sepharose column. After DEAE chromatography, the 30.000MW permeate was analyzed for anti-inflammatory activity.

### Depyrogenation and Analysis of the Endotoxin Content of < 30.000 Dalton Egg Anti-inflammatory Composition Isolates

### Depyrogenation

Egg anti-infiammatory composition isolates were depyrogenated to eliminate the endotoxin which mimics anti-inflammatory composition activity. Briefly, 60 mg of egg anti-inflammatory powder was dissolved in 15 ml of sterile physiological saline solution. The mixture was depyrogenated by ultracentrifugation in 3K MWCO Centriplus Concentrators (Amicon). The Centriplus Concentrators were previously depyrogenated with PyroCLEAN (AlerCHECK. Inc.) using aseptic techniques under laminar flow. Endotoxin may also be removed by standard procedures available to those in the art and need not be based on molecular weight separation.

### Analysis

For verification purposes, depyrogenated egg anti-inflammatory composition was assayed for endotoxin content by the Tumor Necrosis Factor (TNF) enzyme-linked immunosorbent assay (ELISA). Briefly, an aliquot of egg anti-inflammatory composition diluted with physiological saline was added to plates containing J774 macrophages grown in Roswell Park Memorial Institute (RPMI) media with 10% fetal calf serum. After 4 hours of incubation at 37C, the supernatant was added to a plate coated with monoclonal anti-TNF antibody and blocked with 2% bovine serum albumin (BSA) in phosphate-buffered saline (PBS). After overnight incubation of the plate at room temperature, biotinylated antibody and streptavidin peroxidase were added. 3,3',5,5'-tetramethyl benzidine (TMB) substrate was added to the plate for color development. The absorbance of the plate well contents was read at 450 nm. The TNF concentration in the egg anti-inflammatory sample was determined by interpolating from the standard curve. The TNF content of egg anti-inflammatory composition isolates after depyroeenation was negligible. (See Table 3).

**TABLE 3**

| **Endotoxin Content of the Egg Anti-inflammatory Composition Fractions by TNF Assay** | |
|---|---|
| **Fraction** | **pg/ml** |
| S100 Egg Yolk Anti-infiammatory Composition | 98.08 |
| S100 Egg White Anti-inflammatory Composition | 42.00^{a} |
| Non-hyperimmunized Egg Yolk Anti-inflammatory Composition | 66.43^{a} |
| Non-hyperimmunized Egg White Anti-inflammatory Composition | 52.90^{a} |
| Hexane:Ethanol-Extracted Dry Yolk Anti-inflammatory Composition | 92.70^{b} |
| Supercritical CO₂, Extracted Yolk Anti-inflammatory Composition | <18^{b} |

| | |
|---|---|
| ^{a} Media only had 46.5 pg TNF/ml. ^{b} Media had no TNF. | |

### Effect of < 30,000 Dalton Endotoxin-Free Anti-inflammatory Composition on Leukocyte Migration

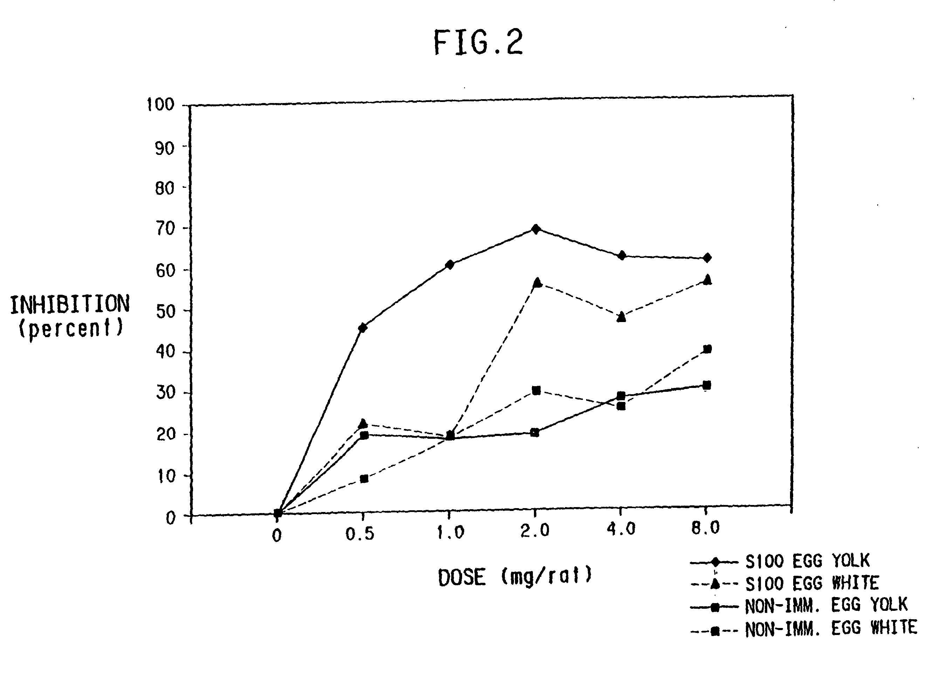

The anti-inflammatory effect of substantially pure <30,000 dalton egg anti-inflammatory composition at each dosage was determined by the reduction in the number of leukocytes in the pleural exudates of substantially pure egg anti-inflammatory composition-treated rats as compared to those of control rats. (See Table 4. Figures 2). Figure 2 and Table 4 compare the effect of the substantially pure hyperimmunized egg yolk and egg white fractions with that obtained from regular table eggs. All doses of substantially pure hyperimmune anti-inflammatory composition resulted in a decrease in the number of leukocytes migrating into the pleural exudate. The greatest effect was observed in those subjects given 2 mg of the substantially pure composition from egg yolk.

**TABLE 4**

| **Inhibition of Leukocyte Migration Into the Pleural Exudates at Different Egg Anti-inflammatory Composition Doses** | | | | |
|---|---|---|---|---|
| | **% Inhibition** | | | |
| **Egg Anti-inflammatory Composition** | **Egg Yolk Anti-inflammatory Composition** | | **Egg White Anti-inflammatory Composition** | |
| Doses | | | | |
| mg/Rat | S100 hyperimmune | Non-hyperimmunized | S100 hyperimmune | Non-hypenmmunized |
| | | | | |
| 0.5 | 45.64 | 18.49 | 22.0 | 8.22 |
| 1.0 | 59.50 | 17.12 | 18.0 | 16.90 |
| 2.0 | 67.20 | 18.72 | 54.4 | 28.31 |
| 4.0 | 61.50 | 26.94 | 46.7 | 23.74 |
| 8.0 | 61.00 | 29.68 | 54.9 | 37.44 |
| | | | | |

### Characterization of Egg Anti-Inflammatory Composition

The molecular weight of the substantially pure egg anti-inflammatory composition prepared by the method described above was found to be less than 30,000 daltons. This was deduced from the fact that the first step in the isolation of the composition from egg yolk and egg white was by ultrafiltration using a membrane that does not allow the passage of molecular weight species >30,000 daltons.

The composition has a negative charge under prevailing conditions, pKa value of this gel is 9.5. This was determined by applying egg ultrafiltrate to a DEAE Sepharose ion exchange column. The anti-inflammatory composition did not elute with water from the column. Changing the elution media to NH₄OAc solution, or any salt solution or buffer with a pH higher than 9.5, caused the egg anti-inflammatory composition to elute. When the ultrafiltrate was applied to the DEAE column, various proteins and salts made up the unbound fraction (Peak 1, Figure 1). Peaks were recorded when the column was washed with NH₄OAc solution (Peak 2) and then NaCl solution (Peak 3). The fraction isolated from Peak 2 exhibited anti-inflammatory activity based on a rat assay. The third peak showed negligible activity. Egg white anti-inflammatory composition is heat-stable at 90 for 1 hr.

Moreover, the egg anti-inflammatory composition can be isolated from egg yolk and egg white. Egg anti-inflammatory composition from egg yolk shows higher activity than egg anti-inflammatory composition from egg white (Figure 2, Table 4).

### Example 4

### Preferred Method for Preparing a Highly Purified Anti-Inflammatory Composition from Egg

The following examples describe a method (suitable for large scale purification) for obtaining the anti-inflammatory composition from avian eggs in its highly pure, lowest molecular weight, non-aggregated form Whole eggs, hyperimmunized and control table eggs, were cracked and the egg white was separated from the yolk and both were spray-dried. Hyperimmunized eggs were obtained as described in Example 1. Egg white powder was processed separately to obtain the aqueous fraction for ultra-filtration.

All of the purification steps were performed so as to minimize possible contamination with bacteria or pyrogens. Sterile water was used to prepare solutions and all glassware was de-pyrogenated. In addition, the solution was sterile filtered.

### Example 4a

### Preparation of Egg Anti-Inflammatory Composition From Egg Yolk Powder Solvent Extraction

The dried egg yolk was subjected to liquid solvent extraction with either propane, or butane to separate the lipids from the aqueous yolk fraction containing the anti-inflammatory composition. Briefly, 500 grams of dry egg yolk powder was placed in a column, to which was added 4 liters of liquid propane solvent. The solvent supernatant and extracted lipid were removed. Six additional solvent extractions were performed for a total of six lipid extractions.

### Ultra-firtration

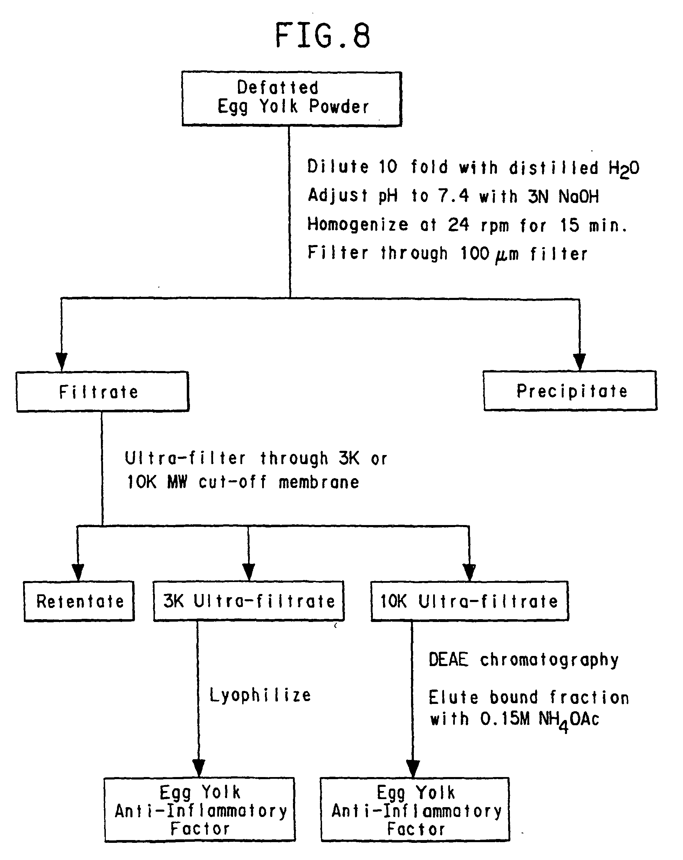

Four hundred grams of dry de-fatted egg yolk was diluted with 4 liters of sterile distilled water and homogenized with a Virtis (handishear). The yolk mixture was either centrifuged at 24 RPM or allowed to stand refrigerated until the non-dissolved yolk particles precipitated. The resulting aqueous fraction was ultrafiltered using an Amicon RA1000 ultra-filtration system equipped with 3,000 MW cut-off spiral-wound membrane. The pump speed was maintained at 20 psi inlet pressure and 15 psi outlet pressure. The <3,000 molecular weight permeates were sterile filtered using a 0.45 µm Sterile disposable Nalgene filter and lyophilized or frozen, for storage, bioassay testing or further purification. Figure 8 shows a flow chart of the purification process.

Molecular species below 3,000 daltons from egg yolk contain the anti-inflammatory composition in a low molecular weight, non-aggregated form. From 400 grams of starting material, the yield of anti-inflammatory composition was approximately 12 grams or 3% of the total. Bioassay testing of this 3K fraction for anti-inflammatory activity showed high levels of activity (example 6a).

### Example 4b

### Preparation of Egg Anti-inflammatory Activity from Egg White Powder

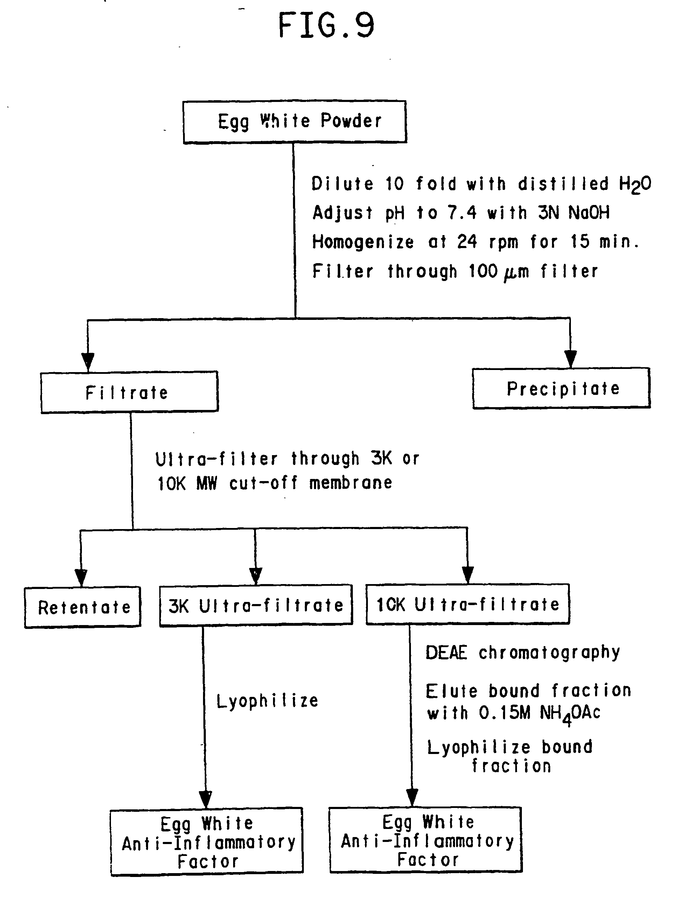

Four grams of egg white, isolated from both hyperimmunized egg as described in Example 1 and control table egg, was diluted with one liter of deionized water. The mixture was homogenized and filtered through a 40 µm filter and ultrafiltered through a 3K MW CO ultra-filtration system (Figure 9). From 400 g of egg white powder, 8.6 g or 2.15 % of anti-inflammatory composition was recovered. This material showed bioassay activity in the inhibition of rat leukocyte migration to an induced site of injury (example 6a).

This example shows the preparation of the active anti-inflammatory composition from egg white material of both hyperimmune and control table eggs.

### Example 5

### DEAE Ion-Exchange Chromatography

The egg anti-inflammatory composition was also further characterized by anion exchange DEAE chromatography. This example demonstrated that the anti-inflammatory composition is negatively charged at neutral pH.

10K ultra-filtrate from both egg white and egg yolk material was obtained in the same manner as described in Example 4a, except that a 10,000 MW membrane was used in place of the 3000 MW membrane

A DEAE - Poros 50 column (10/50 millimeters) was equilibrated with sterile double distilled pyrogen free water (Sigma), pH 7.0. Ten milligrams of the ultra-filtrate (<10.000 MW) dissolved in 100 ml of sterile pyrogen free double distilled water was applied to the DEAE Poros column on the BioCad 700 (Perseptives Biosystems). At a flow rate of 10 ml/min the material was applied to the column. The initial elution of material not bound to the column was removed with distilled water and collected. A linear gradient of ammonium acetate (0% - 100%) was used to elute the material bound to the column and fractions monitored with UV absorption at 280 nm and 254 nm and 10 ml fractions collected by an Avantec fraction collector. NaCl (0.15M) was used to remove the remaining material bound to the column. The column was washed with distilled water until the 280 nm and 254 nm absorbance returned to baseline.

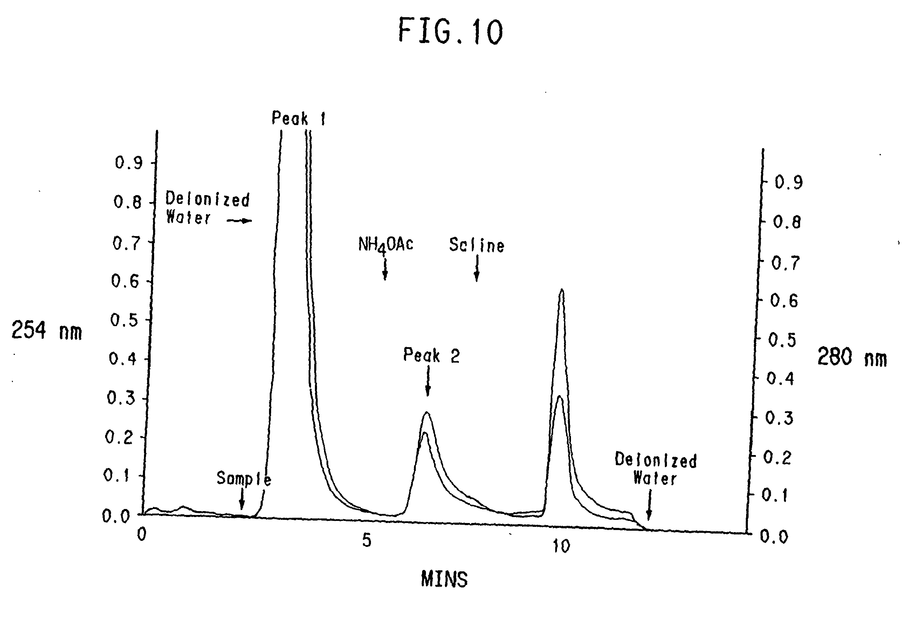

Figure 10 shows a typical chromatogram. The arrow indicates the active anti-inflammatory fraction (peak 2). A typical elution profile with NH₄ Acetate is demonstrated with changes in elution buffer indicated by arrowheads. The eluate was sterile filtered with a 0.45 µm sterile filter, lyophilized to dryness and weighed. Bioassay of the individual peaks showed that the peak containing the activity was found in peak 2 (example 6b). The fraction isolated by DEAE ion exchange chromatography demonstrated that the anti-inflammatory composition was negatively charged.

### Example 6

### Anti-Inflammatory Activity Assay

Blind tests of substantially purified fractions of egg yolk and egg white, from table eggs and hyperimmune eggs were assayed for anti-inflammatory activity. The test assay measured the inhibition of leukocyte migration from venules to an induced site of injury in the pleural cavity of rats.

Female rats (Charles River. MA) weighing 120 to 150 grams were injected into the pleural cavity with 2 ml of 1% Carrageenan (an irritant) in physiological saline to induce leukocyte migration into the pleural cavity. The rats were then injected intra-peritoneally (1P) with 0.5 ml doses of each test fraction in physiological saline at the selected concentration. Two rats were injected at each concentration. An injection of saline was used as a control. Four hours post-iniection, the animals were sacrificed using methods in compliance with the Animal Welfare act. The pleural exudate was removed and the leukocytes were fixed for evaluation. Fifty microliters of the exudate was loaded into a corresponding labeled slide and loaded onto a cytospin. After completion of the cytospin cycle the slides were sprayed with cytoprep fixative (Fisher Scientific Co. Atlanta) and stained with hematoxylin. Five representative areas of each slide were evaluated and the number of leukocytes were counted with the Bioscan Optimas Image Analysis Program. The average of all five counts was used for the total average leukocyte count for each rat. The percent inhibition, compared to control saline injections, was calculated for each sample concentration.

### Example 6a

### Comparison of Anti-inflammatory Activity From Egg White, Egg Yolk, Hyperimmune and Table Eggs.

Purified fractions from spray dried de-fatted egg yolk and egg white from table eggs and hyperimmunized eggs as described in example 4 were tested for anti-inflammatory activity. The less than 3,000 MW permeate from table egg white, hyperimmunized egg yolk and hyperimmunized egg white were obtained after ultra-filtration as described in example 4 and 5. Two animals for each sample were injected IP with either saline, 1, 5, 10, or 20 mg of the test fractions and evaluated as described in example 6.

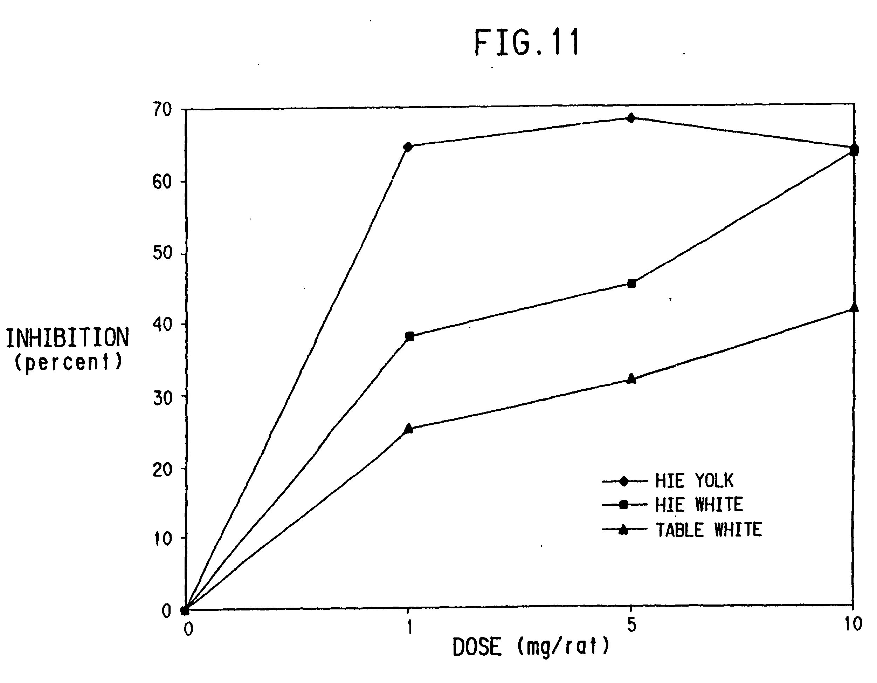

Figure 11 shows that highly purified egg yolk fractions of less than 3,000 molecular weight from hyper-immunized chickens demonstrated a 65% inhibition of leukocyte migration at a dose of 1 mg/rat. These hyperimmune egg yolk fractions also demonstrated a higher level of activity (65% inhibition at a dose of 1mg/rat) than fractions obtained from hyperimmune egg white (38%) at the same dose. Control egg white demonstrated less anti-inflammatory activity (25% inhibition at a dose of 1 mg/rat). Non-hyperimmunized egg fractions also contain the anti-inflammatory activity since all hens are vaccinated and are subject to natural immunization from the presence of microorganisms and allergens in the environment. Surprisingly however, the level of the anti-inflammatory composition was increased in both yolk and white by the process of hyperimmunization. Since components in egg are generally compartmentalized either into the lipid rich yolk or white, it is surprising that the anti-inflammatory activity was found in the less than 3,000 MW fraction from both egg yolk and egg white.

This example shows that the anti-inflammatory composition is less than 3,000 daltons, is found in egg yolk and egg white and is found in much higher and effective amounts in hyperimmune egg fractions than in control table eggs. In fact, the highly pure anti-inflammatory composition is found in hyperimmunized eggs in an amount which is effective in treating inflammation.

### Example 6b

### Effect of the Negatively Charged DEAE Fractions on Leukocyte Migration

Following the anti-inflammatory bioassay procedure, the negatively charged DEAE peak 2 eluted with NH₄ OAc the 3K and 10K ultra-filtrates from hyperimmune egg yolk were tested for activity. Each sample was tested at the doses indicated.

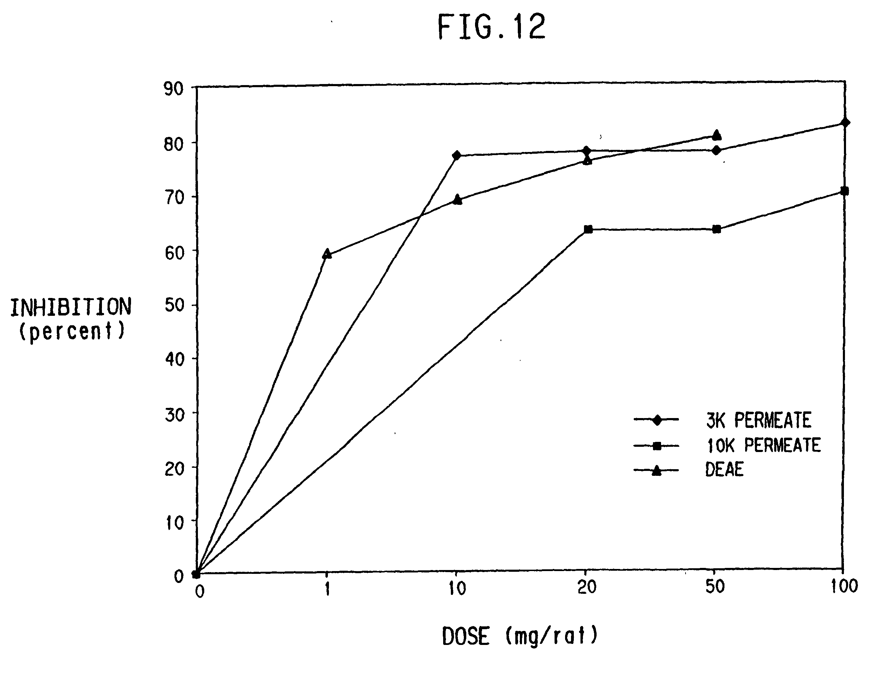

Figure 12 shows that the negatively charged DEAE fraction, the 3K and 10K ultra-filtrate of eggs from hyperimmunized chickens show a high level of anti-inflammatory activity when compared with the saline control.

### Example 6c

### Comparison With Anti-Inflammatory Drugs

The anti-inflammatory drugs indomethacin and aspirin were compared with the egg 3K ultra-filtrate for inhibition of leukocyte migration in vivo. In this assay, intra-peritoneal injections were given at the doses of : 1000µg, 100µg, 10µg, 1µg, for indomethacin, aspirin and the 3K egg anti-inflammatory composition from hyperimmune egg.

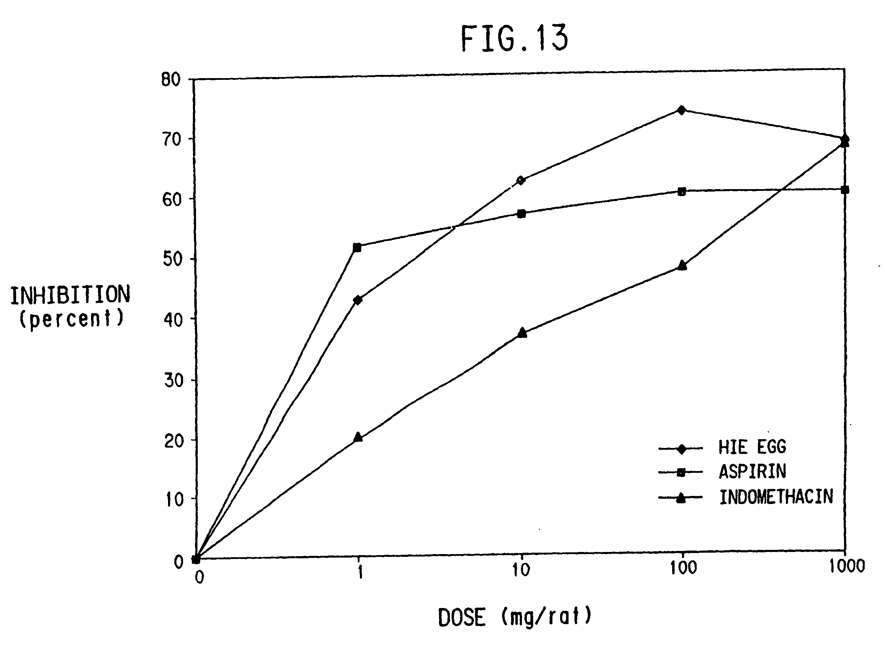

Figure 13 shows that the 3K ultra-filtrate and aspirin showed similar levels of anti-inflammatory activity. The level of inhibition of leukocyte migration by indomethacin appears to be reduced at lower doses (1 - 100 µg/rat).

This example shows that the anti-inflammatory fractions from egg has an effect comparable in the leukocyte migration assay to known anti-inflammatory drugs.

### Example 7

### Purification of Anti-Inflammatory Factor by Reverse Phase High Pressure Liquid Chromatography (HPLC)

Substantially pure less than 3K. 10K DEAE fractions from egg yolk and 30K DEAE fraction from egg white were separated on a Waters C8 symmetry reverse phase column (3.9 x 250 mm). A linear gradient of 10% - 80% methanol and H₂O was used as the mobile phase on a Waters Model 2010 HPLC with a 717 plus auto-sampler and a 996 photo-diode array detector. The lyophilized samples were diluted in 100 to 200 ml of sterile double distilled water. The column was eluted at a flow rate of 2 ml per minute and the effluent diode array data was stored at all wavelengths from 200 nm to 350 nm.

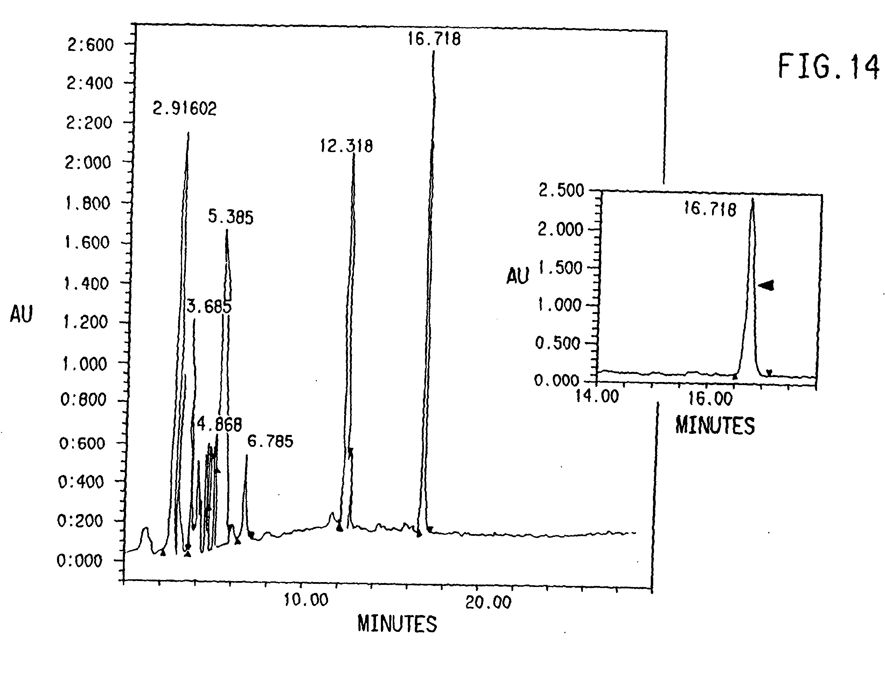

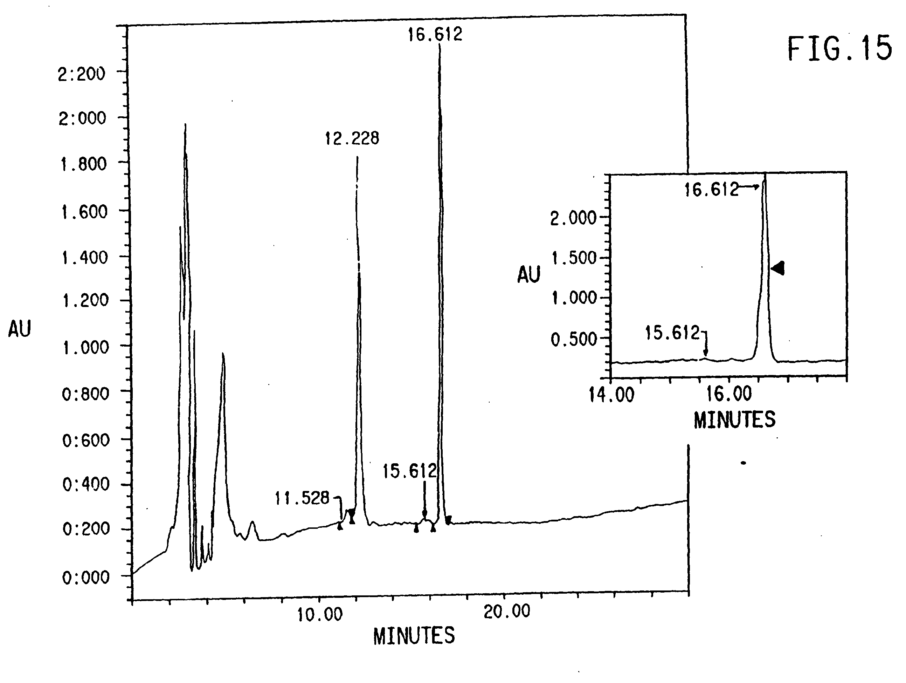

Figure 14 shows a MAX plot chromatogram of the separation of the 3K permeate. This chromatogram shows the two major peaks of less than 3K molecular weight that result in a highly purified egg anti-inflammatory composition. Figure 15 illustrates the separation of the composition from a 10K permeate which was separated on a DEAE ion exchange column as outlined in example 5. Two peaks similar to those observed with the 3K ultra-filtrate fraction were obtained. These results indicate that both fractions, less than 3K and DEAE which also showed anti-inflammatory activity (example 7), can be used to obtain a highly purified negatively charged component.

Analysis of the composition of 3K egg yolk fraction showed the presence of an initial series of peaks eluting at one to two minutes and three peaks eluting at 10 to 20 minutes. This example shows that the anti-inflammatory composition is less than 3,000 MW and can be separated by the method into a relatively pure peak.

### Preparative HPLC

To obtain large quantities of the anti-inflammatory factor after ultrafiltration, the preferred step is to separate the highly purified, less than 3,000 molecular weight, composition on a preparative HPLC column. Accordingly, a 5.08 X 20 cm column, was packed with Zorbax C8 packing material and 4 grams of the less than 3,000 MW ultrafiltrate was purified during each run. Using a mobile phase of 80/20 H₂O/methanol with 0.05% TFA, at a flow rate of 90 ml per minute, the permeate was separated into the highly purified composition collected and lyophilize. Next the collected peak was re-tested on an analytical C8 column. With the method described, large quantities of the ultra-pure peak were obtained by preparative HPLC and analyzed for bioassay activity, elemental composition and chemical structure. Mass Spectroscopy. Elemental analysis. Infra-red spectroscopy and Nuclear Magnetic Resonance. NMR are being used to determine the structure of the factor.

### Example 8

### Physical Characteristics Of Anti-Inflammatory Composition From Egg.

This example illustrates the stability to heating, changes in acid and base, proteinase treatment.

Four milligrams of the less than 3,000 MW ultra-filtrate was diluted in sterile water and subjected to 100 °C for 30 minutes. Samples of the less than 3K ultra-filtrate were subjected to acid HCL pH 2.0, neutral pH 7.4 or base NaOH pH 9.0, for 30 minutes before neutralizing. 3K fractions were also treated with 1 unit of pronase enzyme for one hour. All samples were sterile filtered, lyophilized, weighed and tested for biological activity.

Comparisons of the bioassay activity of the treated fractions (high heat, changes in pH, and pronase digestion) with the untreated control and saline injected animals show a surprising stability to these treatments. Stability to high heat, changes in pH, and pronase treatment in addition to the small size (less than 3.000 MW) indicate that the anti-inflammatory composition is not protein.

It was determined that the highly pure anti-inflammatory composition has the following characteristics. It has a molecular weight of less than 3,000 dalton. This characteristic is deduced from the isolation and purification of the composition wherein the isolation and purification process uses an ultra-filtration membrane that does not allow the passage of molecular species greater than 3,000 dalton there through. The composition is determined to be non-proteinaceous and non-steroidal because it is small in size and is not degraded by enzymes which degrade proteins. Moreover, the composition is orally active and is not degraded by digestive enzymes. The small stable form of the composition (as differentiated from proteins which are much larger) facilitates its absorption from the digestive tract.

The composition is acid and base resistant, reverting to an active form even after treatment with conditions of pH 9.0. In addition, the composition is heat-stable at 100C for at least 30 minutes and has a negative charge at neutral pH.

The egg anti-inflammatory composition can be isolated from whole egg, egg yolk and egg white. Anti-infiammatory composition isolated from egg yolk shows higher anti-inflammatory activity than anti-inflammatory composition purified from egg white.

Supra-normal levels of the anti-inflammatory composition can be isolated from hyperimmune whole egg, hyperimmune egg yolk and hyperimmune egg-white.

## Claims

1. A method for highly purifying an anti-inflammarory composition from an egg of an egg-producing animal comprising:
(1) isolating a water soluble fraction from the whole egg, egg yolk or egg white;
(2) separating a less than 3,000 dalton permeate from the water soluble fraction;
(3) fractionating said less than 3,000 dalton permeate to recover a biologically active fraction.

2. The method of claim 1 wherein said fractionating step further comprises reverse phase high pressure liquid chromatography of said less than 3.000 dalton permeate.

3. The method of claim 1 further comprising de-fatting the whole egg or the egg yolk prior to step (1).

4. The method of claim 1 further comprising lyophilizing said < 3,000 dalton permeate of step (2).

5. The method of claim 1 wherein the separating step further comprises ultrafiltering the water soluble fraction through a filter with a molecular weight cut-off of 3,000 daltons.

6. The method of claim 1 wherein the egg-producing animal is maintained in a hyperimmunized state.

7. The method of claim 6 wherein the hyperimmunized state is induced by an antigenic, genetic, or bioengineered vaccine.

8. The method of claim 7 wherein the genetic vaccine comprises at least one antigen coding DNA construct selected from the group consisting essentially of fragments of naked DNA, plasmid DNA, viral DNA, DNA expression libraries. DNA-RNA antigens. DNA-protein conjugates and DNA liposome conjugates and combinations thereof.

9. The method of claim 7 wherein the antigenic vaccine comprises at least one antigen selected from the group consisting of bacterial, viral, protozoan, fungal and cellular antigens.

10. An ami-inflammatory composition, in highly purified form, obtainable at supranormal levels from an egg of an egg producing animal maintained on a hyperimmunized state by a process comprising:
(1) isolating a water soluble fraction from the whole egg, egg yolk or egg white;
(2) separating a less than 3.000 dalton permeate from the water soluble fraction;
(3) fractionating said less than 3.000 dalton permeate to recover supranormal levels of said auto-inframmatory composition.

11. The anti-inflammatory composition of claim 10 wherein the egg-producing animal is in a hyperimmunized state.

12. The anti-inflammatory composition of claim 10 wherein the hyperimmunized state is induced by an antigenic, genetic, or bioengineered vaccine.

13. The anti-inflammatory composition of claim 12 wherein the genetic vaccine comprises at least one antigen coding DNA construct selected from the group consisting essentially of fragments of naked DNA, plasmid DNA, viral DNA, DNA expression libraries, DNA-RNA antigens, DNA-protein conjugates and DNA liposome conjugates, and combinations thereof.

14. The anti-inflammatory composition of claim 12 wherein the antigenic vaccine comprises at least one antigen selected from the group consisting of bacterial, viral, protozoan, fungal and cellular antigens and combinations thereof.

15. Use of an anti-inflammatory composition according to any one of claims 10 to 14 in the manufacture of a medicament for preventing, countering or reducing inflammation

16. The use of claim 15 wherein the medicament comprises an amount of the anti-inflammatory composition in the range from 5 micrograms to 300 milligrams.

17. Use of an anti-inflammatory composition according to any one of claims 10 to 14 in the manufacture of a medicament for inhibiting leukocyte migration.

## Patentansprüche

1. Verfahren zum hohen Reinigen einer entzündungshemmenden Zusammensetzung aus einem Ei eines Ei-produzierenden Tieres, umfassend:
(1) das Isolieren einer wasserlöslichen Fraktion aus dem Vollei, Eigelb oder Eiweiß,
(2) das Trennen eines Permeats mit weniger als 3000 Dalton von der wasserlöslichen Fraktion,
(3) das Fraktionieren des Permeats mit weniger als 3000 Dalton, um eine biologisch aktive Fraktion zu gewinnen.

2. Verfahren nach Anspruch 1, wobei der Fraktionierungsschritt weiter Umkehrphasen-Hochdruck-Flüssigchromatographie des Permeats mit weniger als 3000 Dalton umfasst.

3. Verfahren nach Anspruch 1, weiter umfassend das Entfetten des Volleis oder des Eigelbs vor Schritt (1).

4. Verfahren nach Anspruch 1, weiter umfassend das Lyophilisieren des < 3000 Dalton Permeats von Schritt (2).

5. Verfahren nach Anspruch 1, wobei der Trennschritt weiter das Ultrafiltrieren der wasserlöslichen Fraktion durch einen Filter mit einem Cut-off-Molekulargewicht von 3000 Dalton umfasst.

6. Verfahren nach Anspruch 1, wobei das Ei-produzierende Tier in einem hyperimmunisierten Zustand gehalten wird.

7. Verfahren nach Anspruch 6, wobei der hyperimmunisierte Zustand durch einen antigenischen, genetischen oder bioverfahrenstechnischen Impfstoff induziert wird.

8. Verfahren nach Anspruch 7, wobei der genetische Impfstoff mindestens ein Antigen-kodierendes DNA-Konstrukt umfasst, welches aus der Gruppe, im Wesentlichen bestehend aus Fragmenten nackter DNA, Plasmid-DNA, viraler DNA, DNA-Expressionsbibliotheken, DNA-RNA-Antigenen, DNA-Protein-Konjugaten und DNA-Liposom-Konjugaten und Kombinationen davon, ausgewählt ist.

9. Verfahren nach Anspruch 7, wobei der antigenische Impfstoff mindestens ein Antigen, ausgewählt aus der Gruppe, bestehend aus bakteriellen, viralen, protozoischen, fungalen und zellulären Antigenen, umfasst.

10. Entzündungshemmende Zusammensetzung in hochreiner Form, erhältlich in übernormalen Niveaus aus einem Ei eines Ei-produzierenden Tiers, welches in einem hyperimmunisierten Zustand gehalten wird, durch ein Verfahren, umfassend:
(1) das Isolieren einer wasserlöslichen Fraktion aus dem Vollei, Eigelb oder Eiweiß,
(2) das Trennen eines Permeats mit weniger als 3000 Dalton von der wasserlöslichen Fraktion,
(3) das Fraktionieren des Permeats mit weniger als 3000 Dalton, um übernormale Niveaus der entzündungshemmenden Zusammensetzung zu gewinnen.

11. Entzündungshemmende Zusammensetzung nach Anspruch 10, wobei das Ei-produzierende Tier in einem hyperimmunisierten Zustand ist.

12. Entzündungshemmende Zusammensetzung nach Anspruch 10, wobei der hyperimmunisierte Zustand durch einen antigenischen, genetischen oder bioverfahrenstechnischen Impfstoff induziert ist.

13. Entzündungshemmende Zusammensetzung nach Anspruch 12, wobei der genetische Impfstoff mindestens ein Antigen-kodierendes DNA-Konstrukt umfasst, welches aus der Gruppe, im Wesentlichen bestehend aus Fragmenten nackter DNA, Plasmid-DNA, viraler DNA, DNA-Expressionsbibliotheken, DNA-RNA-Antigenen, DNA-Protein-Konjugaten und DNA-Liposom-Konjugaten und Kombinationen davon, ausgewählt ist.

14. Entzündungshemmende Zusammensetzung nach Anspruch 12, wobei der antigenische Impfstoff mindestens ein Antigen umfasst, welches aus der Gruppe, bestehend aus bakteriellen, viralen, protozoischen, fungalen und zellulären Antigenen und Kombinationen davon, ausgewählt ist.

15. Verwendung einer entzündungshemmenden Zusammensetzung nach einem der Ansprüche 10 bis 14 in der Herstellung eines Medikaments zum Vorbeugen, Entgegenwirken oder Reduzieren einer Entzündung.

16. Verwendung nach Anspruch 15, wobei das Medikament eine Menge der entzündungshemmenden Zusammensetzung im Bereich von 5 µg bis 300 µg umfasst.

17. Verwendung einer entzündungshemmenden Zusammensetzung nach einem der Ansprüche 10 bis 14 in der Herstellung eines Medikaments zur Hemmung von Leukozytenmigration.

## Revendications

1. Procédé pour hautement purifier une composition anti-inflammatoire issue d'un oeuf d'un animal produisant des oeufs, comprenant les étapes consistant à :
(1) isoler une fraction hydrosoluble de l'oeuf entier, du jaune d'oeuf ou du blanc d'oeuf ;
(2) séparer un produit de perméation de moins de 3 000 daltons de la fraction hydrosoluble ;
(3) fractionner ledit produit de perméation de moins de 3 000 daltons pour récupérer une fraction biologiquement active.

2. Procédé selon la revendication 1, dans lequel ladite étape de fractionnement comprend, en outre, une chromatographie liquide à haute pression en phase inverse dudit produit de perméation inférieur à 3 000 daltons.

3. Procédé selon la revendication 1, comprenant, en outre, le dégraissage de l'oeuf entier ou du jaune d'oeuf avant l'étape (1).

4. Procédé selon la revendication 1, comprenant, en outre, la lyophilisation dudit produit de perméation de moins de 3 000 daltons de l'étape (2).

5. Procédé selon la revendication 1, dans lequel l'étape de séparation comprend, en outre, l'ultrafiltration de la fraction hydrosoluble à travers un filtre présentant un seuil de rétention du poids moléculaire de 3 000 daltons.

6. Procédé selon la revendication 1, dans lequel l'animal produisant un oeuf est maintenu dans un état hyperimmunisé.

7. Procédé selon la revendication 6, dans lequel l'état hyperimmunisé est induit par un vaccin antigénique, génétique ou produit par génie génétique.

8. Procédé selon la revendication 7, dans lequel le vaccin génétique comprend au moins une construction d'ADN codant l'antigène sélectionnée dans le groupe se composant essentiellement de fragments d'ADN nu, d'ADN plasmide, d'ADN viral, de bibliothèques d'expression d'ADN, d'antigènes ADN-ARN, de conjugués ADN-protéine, de conjugués liposome-ADN et des combinaisons de ceux-ci.

9. Procédé selon la revendication 7, dans lequel le vaccin antigénique comprend au moins un antigène sélectionné dans le groupe consistant en des antigènes bactériens, viraux, protozoaires, fongiques et cellulaires.

10. Composition anti-inflammatoire, sous une forme éminemment purifiée, pouvant être obtenue à des niveaux supranormaux à partir d'un oeuf d'un animal produisant des oeufs maintenu dans un état hyperimmunisé par un procédé comprenant les étapes consistant à
(1) isoler une fraction hydrosoluble de l'oeuf entier, du jaune d'oeuf ou du blanc d'oeuf ;
(2) séparer un produit de perméation de moins de 3 000 daltons de la fraction hydrosoluble ;
(3) fractionner ledit produit de perméation de moins de 3 000 daltons pour récupérer des niveaux supranormaux de ladite composition anti-inflammatoire.

11. Composition anti-inflammatoire selon la revendication 10, dans laquelle l'animal produisant des oeufs se trouve dans un état hyperimmunisé.

12. Composition anti-inflammatoire selon la revendication 10, dans laquelle l'état hyperimmunisé est induit par un vaccin antigénique, génétique ou produit par génie génétique.

13. Composition anti-inflammatoire selon la revendication 12, dans laquelle le vaccin génétique comprend au moins une construction d'ADN codant l'antigène sélectionnée dans le groupe se composant essentiellement de fragments d'ADN nu, d'ADN plasmide, d'ADN viral, de bibliothèques d'expression d'ADN, d'antigènes ADN-ARN, de conjugués ADN-protéine, de conjugués liposome-ADN et des combinaisons de ceux-ci.

14. Composition anti-inflammatoire selon la revendication 12, dans laquelle le vaccin antigénique comprend au moins un antigène sélectionné dans le groupe consistant en des antigènes bactériens, viraux, protozoaires, fongiques et cellulaires et des combinaisons de ceux-ci.

15. Utilisation d'une composition anti-inflammatoire selon l'une quelconque des revendications 10 à 14 dans la préparation d'un médicament destiné à prévenir, contrer ou réduire l'inflammation.

16. Utilisation selon la revendication 15, dans laquelle le médicament comprend une quantité de la composition anti-inflammatoire s'inscrivant dans une plage allant de 5 microgrammes à 300 milligrammes.

17. Utilisation d'une composition anti-inflammatoire selon l'une quelconque des revendications 10 à 14 dans la préparation d'un médicament destiné à inhiber la migration des leucocytes.
